# EUROPEAN PATENT APPLICATION

(11) **EP 2 184 278 A1**
(43) Date of publication of application: **12.05.2010**
(21) Application number: 08792488.2
(22) Date of filing: 08.08.2008
(51) Int. Cl.: C07D 241/04, A61K 31/495, A61K 31/496, A61P 25/04, A61P 29/02, A61P 43/00, C07D 405/12

(54) **P2X4 RECEPTOR ANTAGONIST**

(30) Priority: 10.08.2007 JP 2007209136
(71) Applicant: Nippon Chemiphar Co., Ltd., Chiyoda-ku Tokyo 101-0032 (JP)
(72) Inventor: SAKUMA, Shogo, Misato-shi Saitama 341-0005 (JP); ENDO, Tsuyoshi, Misato-shi Saitama 341-0005 (JP); KANAKUBO, Noriko, Misato-shi Saitama 341-0005 (JP); ARAI, Masahiko, Misato-shi Saitama 341-0005 (JP); TAKAHASHI, Toshihiro, Misato-shi Saitama 341-0005 (JP); IMAI, Toshiyasu, Misato-shi Saitama 341-0005 (JP); TAGUCHI, Kumiko, Misato-shi Saitama 341-0005 (JP); NAKATA, Eriko, Misato-shi Saitama 341-0005 (JP); MOCHIDUKI, Nobutaka, Misato-shi Saitama 341-0005 (JP); USHIODA, Masatoshi, Misato-shi Saitama 341-0005 (JP); TSUDA, Makoto, Fukuoka-shi Fukuoka 812-8581 (JP); INOUE, Kazuhide, Fukuoka-shi Fukuoka 812-8581 (JP)
(74) Representative: Albrecht, Thomas
(86) International application number: PCT/JP2008/064611
(87) International publication number: WO 2009/022731

(57) **Abstract**

A P2X₄ receptor antagonist is a compound represented by the formula (I) or a pharmacologically acceptable salt thereof, which is used as a preventive or therapeutic agent for neuropathic pains: 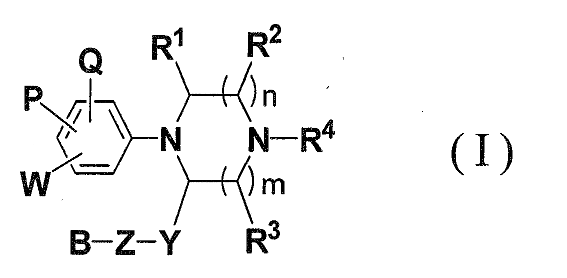 Wherein B is aryl or the like that can have a substituent; Y is C₁₋₅ alkylene that can have a double bond; Z is O, S or the like; each of R¹, R² and R³ independently is hydrogen, C₁₋₈ alkyl or the like; R⁴ is hydrogen, C₁₋₈ alkyl or the like; each of P and Q independently is hydrogen, halogen, C₁₋₈ alkyl or the like; W is C₁₋₈ alkyl or the like; and each of n and m independently is 1 or 2.

## Description

### Field of the invention

The present invention relates to phenylpiperazine derivatives showing P2X₄ receptor antagonism.

### Background of the invention

ATP receptors are basically classified into P2X family of ion-channel type receptors and P2Y family of G protein-coupled receptors. Until now, there are reported, respectively, seven sub-types (P2X₁-₇) and eight sub-types (P2Y_{1, 2, 4, 6, 11-14}).

It has been reported that P2X₄ receptor (Genebank No. X87763), which is a sub-type of P2X family, is present widely in the central nervous systems:
Non-patent document 1: Buel1 et al. (1996) EMBO J. 15: 55-62;
Non-patent document 2: Seguela et al. (1996) J. Neurosci. 16: 448-455;
Non-patent document 3: Bo et al. (1995) FEBS Lett. 375: 129-133;
Non-patent document 4: Soto et al. (1996) Proc. Natl. Acad. Sci. USA 93: 3684-3788;
Non-patent document 5: Wang et al. (1996) Biochem. Res. Commun. 220: 196-202.

The mechanism of pathogenesis of intractable pains such as neuropathic pain is unclear. Therefore, if non-steroidal anti-inflammatory drugs (NSAIDs) and morphine are less effective in patients, there is no other way of pharmacotherapy. In that case, the patient and surrounding people take up a heavy burden in mind and body. The neuropathic pain is caused by injury of peripheral or central nervous systems, for instance, post-surgery pain, spinal cord injury, herpes zoster, or trigeminal neuralgia.

Recently, Inoue, et al. examined the involvement of P2X receptors in neuropathic pain using dorsal root ganglion neuron-injured animal model which induces allodynia, and suggested that the nerve-injured pain (particularly, allodynia) is caused via P2X₄ receptors on spinal microglia:
Non-patent document 6: M. Tsuda et al. (2003) Nature, 424, 778-783;
Non-patent document 7: Jeffrey A.M. Coull et al. (2005) Nature, 438, 1017-1021; and
Patent document 1: United States patent publication No. 20050074819.

Accordingly, compounds enabling to inhibit the action of P2X₄ receptors are expected to be employed for preventing or treating neuropathic pains.

WO 2004/085440 (Patent document 2) discloses that benzofuro-1,4-diazepin-2-one derivatives having the below-illustrated formula (A) show P2X₄ receptor antagonism: 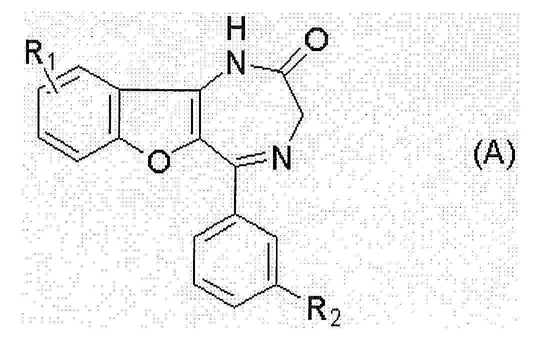 in which R₁ is halogen, and R₂ is hydrogen, halogen, nitro, cyano, C(O)-OR₃, C(O)-NR₄R₅, SO₂-OR₃, or SO₂-NR₄R₅, or in which R¹ is hydrogen, and R₂ is halogen, nitro, cyano, C(O)-OR₃, C(O)-NR₄R₅, SO₂-OR₃, or SO₂-NR₄R₅.

WO 2007/049825 (Patent document 3) discloses that a tricyclic antidepressant such as imipramine shows P2X₄ receptor antagonism.

Abstract P3-N-114 of the 49th Meeting of the Neurochemical Society of Japan (Non-patent document 8) discloses that paroxetine, another antidepressant shows P2X₄ receptor antagonism.

It has been known that SSRI such as paroxetine shows fewer serious side effects in monotherapy. It is true in the case of comparison with conventionally used tricyclic antidepressants. On the other hand, the occurrences of milder side effects are not few. A serious side effect on central nervous system is an increase in the suicidal risk. In 1990 or thereabout, it was reported in United States that SSRI caused strong suicidality. Suits have been frequently filed about the suicides caused by SSRI. Ministry of Health, Labour and Welfare (Japan) instructs that the description "Since an increased risk of suicidality has been reported in young adults, administration should be followed with careful observation." should be added to the document attached to paroxetine. A separation between the effects of SSRI and its analgesic functions can isolate the increased risk of suicidality, which can be a side effect causing a social problem, to obtain safer analgesic functions.

The present inventors have filed WO 2007/072974 (Patent document 4), WO 2007/074940 (Patent document 5), and WO 2008/023847 (Patent document 6), each of which discloses tricyclic compounds showing P2X₄ receptor antagonism.

WO 2005/037803 (Patent document 7) and WO 2004/089915 (Patent document 8) disclose the compounds (B), (C) or the like showing a function of inhibiting renin.

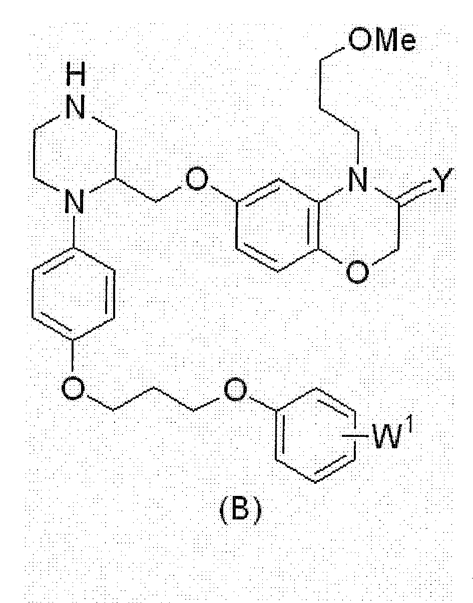

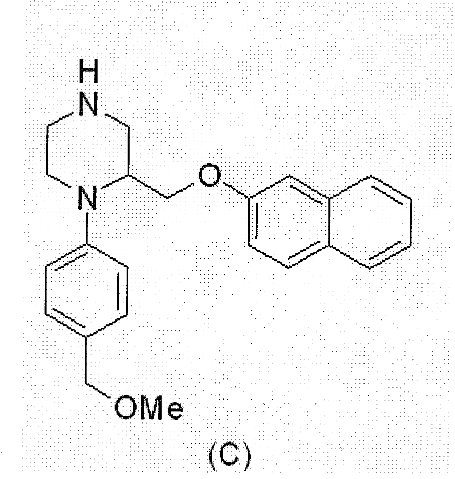

The documents disclose neither P2X₄ receptor antagonism nor analgesic effect on neuropathic pain about the compounds (B) and (C).

### Disclosure of the invention

The object of the present invention is to provide a piperazine derivative represented by the formula (I), and a P2X₄ receptor antagonist or a preventive or therapeutic agent for neuropathic pains, which contains it as an active ingredient.

The present invention relates to a compound represented by the formula (I) or its pharmacologically acceptable salt: 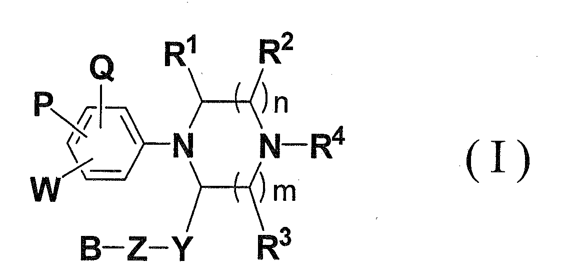 wherein B represents an aryl group or a heterocyclic group, each of which can have a substituent;
Y represents alkylene having 1 to 5 carbon atoms, which can have a double bond;
Z represents O, S, N(R⁵), or a chemical bond, wherein R⁵ represents a hydrogen atom or an alkyl group having 1 to 8 carbon atoms;
each of R¹, R², and R³ independently represents a hydrogen atom, an alkyl group having 1 to 8 carbon atoms, or a haloalkyl group having 1 to 8 carbon atoms and 1 to 3 halogen atoms;
R⁴ represents a hydrogen atom, an alkyl group having 1 to 8 carbon atoms, a haloalkyl group having 1 to 8 carbon atoms and 1 to 3 halogen atoms, a three- to seven-membered cycloalkyl group, or an alkyl group having 1 to 8 carbon atoms substituted with a three- to seven-membered cycloalkyl;
each of P and Q independently represents a hydrogen atom, a halogen atom, a alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, or a heterocyclic group;
W represents an alkyl group having 1 to 8 carbon atoms or a three- to seven-membered cycloalkyl group, or P and W are combined to form propylene or tetramethylene in the case that P and W are placed at the 2- and 3-positions or the 3- and 4-positions of the phenyl group; and
each of n and m independently represents 1 or 2.

The invention also relates to a P2X₄ receptor antagonist containing a compound represented by the formula (I) or its pharmacologically acceptable salt as an active ingredient.

The invention further relates to a preventive or therapeutic agent for neuropathic pains containing a compound represented by the formula (I) or its pharmacologically acceptable salt as an active ingredient.

### The best mode of the invention

The present invention is described below in more detail.

The compound represented by the formula (I) or its pharmacologically acceptable salt preferably is the following compound or its pharmacologically acceptable salt in the present invention.
(1) A compound represented by the formula (I) or its pharmacologically acceptable salt, wherein B represents phenyl, naphthyl, benzofuranyl, indolyl, benzothienyl, or thienyl, each of which can have one to three substituent groups selected from the group consisting of a halogen atom, an alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, an aryloxy group having 6 to 12 carbon atoms, an arylalkyloxy group comprising an alkyl moiety having 1 to 8 carbon atoms, an alkoxycarbonyl group having 2 to 9 carbon atoms, a carbamoyl group, an alkylcarbamoyl group having 2 to 9 carbon atoms, a sulfamoyl group, an alkylsulfamoyl group having 1 to 8 carbon atoms, and a dialkylsulfamoyl group having 2 to 16 carbon atoms.
(2) A compound represented by the formula (I) or its pharmacologically acceptable salt, wherein B represents phenyl, which can have one to three substituent groups selected from the group consisting of a halogen atom, an alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, an aryloxy group having 6 to 12 carbon atoms, an arylalkyloxy group comprising an alkyl moiety having 1 to 8 carbon atoms, an alkoxycarbonyl group having 2 to 9 carbon atoms, a carbamoyl group, an alkylcarbamoyl group having 2 to 9 carbon atoms, a sulfamoyl group, an alkylsulfamoyl group having 1 to 8 carbon atoms, and a dialkylsulfamoyl group having 2 to 16 carbon atoms.
(3) A compound represented by the formula (I) or described in (1), (2), or its pharmacologically acceptable salt, wherein each of P and Q independently represents a hydrogen atom, a alkyl group having 1 to 8 carbon atoms, a haloalkyl group having 1 to 8 carbon atoms and 1 to 3 halogen atoms, or an alkoxy group having 1 to 8 carbon atoms.
(4) A compound represented by the formula (I) or described in one of (1) to (3), or its pharmacologically acceptable salt, wherein each of P and Q represents a hydrogen atom.
(5) A compound represented by the formula (I) or described in one of (1) to (4), or its pharmacologically acceptable salt, wherein W represents an alkyl group having 3 to 6 carbon atoms.
(6) A compound represented by the formula (I) or described in one of (1) to (4), or its pharmacologically acceptable salt, wherein W represents n-propyl, isopropyl, n-butyl, or isobutyl.
(7) A compound represented by the formula (I) or described in one of (1) to (6), or its pharmacologically acceptable salt, wherein each of n and m represents 1.
(8) A compound represented by the formula (I) or described in one of (1) to (7), or its pharmacologically acceptable salt, wherein Y represents methylene.
(9) A compound represented by the formula (I) or described in one of (1) to (8), or its pharmacologically acceptable salt, wherein Z represents O or S.
(10) A compound represented by the formula (I) or described in one of (1) to (9), or its pharmacologically acceptable salt, wherein each of R¹, R², and R³ represents a hydrogen atom.
(11) A compound represented by the formula (I) or described in one of (1) to (10), or its pharmacologically acceptable salt, wherein R⁴ represents a hydrogen atom or an alkyl group having 1 to 8 carbon atoms.
(12) A compound represented by the formula (I) or described in one of (1) to (10), or its pharmacologically acceptable salt, wherein R⁴ represents a hydrogen atom.
(13) A compound represented by the formula (I) or its pharmacologically acceptable salt, wherein R⁴ represents a hydrogen atom, Y represents methylene, Z represents O or S, and B represents phenyl, which can have one to three substituent groups selected from the group consisting of a halogen atom, an alkyl group having 1 to 8 carbon atoms, a haloalkyl group having 1 to 8 carbon atoms and 1 to 3 halogen atoms, a cyano group, a hydroxyl group, an alkoxy group having 1 to 8 carbon atoms, a haloalkoxy group having 1 to 8 carbon atoms and 1 to 3 halogen atoms, a benzyloxy group, a sulfamoyl group, and an alkylsulfamoyl group having 1 to 8 carbon atoms.
(14) A compound selected from the group consisting of 2-(4-chlorophenoxymethyl)-1-(4-isopropylphenyl)-piperazine, 2-(4-chlorophenoxymethyl)-1-(4-propylphenyl)-piperazine, 2-(4-chlorophenoxymethyl)-1-(3-isopropylphenyl)piperazine, 2-(4-chlorophenoxymethyl)-1-(2,4,6-trimethylphenyl)piperazine, 2-(4-chlorophenoxymethyl)-(1-indan-5-yl)piperazine, 1-(4-isopropylphenyl)-2-[4-(isopropylsulfamoyl)phenoxymethyl]piperazine, 2-(4-chlorophenylsulfanylmethyl)-1-(4-isopropylphenyl)piperazine, 1-(3-isopropylphenyl)-2-[4-(isopropylsulfamoyl)phenoxymethyl]piperazine, and 1-(4-isopropylphenyl)-2-(4-phenoxyphenoxymethyl)piperazine or its pharmacologically acceptable salt.

In the present specification, the aryl group can be phenyl and naphthyl.

The heterocyclic group can be benzofuranyl, 1,3-benzo[d]dioxolyl, quinolyl, indolyl, benzothienyl, thienyl, or pyridyl.

The alkylene having 1 to 5 carbon atoms can be alkylene having 1 to 3 carbon atoms such as propylene, ethylene, methylene, and preferably is methylene.

The alkylene having 1 to 5 carbon atoms and a double bond can be -CH₂CH=CH-.

The alkyl group having 1 to 8 carbon atoms can be methyl, ethyl, propyl, isopropyl, butyl, isobutyl, t-butyl, pentyl, or hexyl.

The haloalkyl group having 1 to 8 carbon atoms and 1 to 3 halogen atoms can be methyl, ethyl, propyl, isopropyl, butyl, or t-butyl substituted with 1 to 3 halogen atoms such as a fluoro atom, a chloro atom, or a bromo atom, and preferably is trifluoromethyl, chloromethyl, 2-chloroethyl, 2-bromoethyl, or 2-fluoroethyl.

The three- to seven-membered cycloalkyl group can be cyclohexyl or cyclopentyl.

The alkyl group having 1 to 8 carbon atoms substituted with a three- to seven-membered cycloalkyl can be cyclopropylmethyl.

The halogen atom can be a fluoro atom, a chloro atom, or a bromo atom.

The alkylamino group having 1 to 8 carbon atoms can be methylamino or ethylamino.

The dialkylamino group having 2 to 16 carbon atoms can be dimethylamino or diethylamino.

The aryloxy group having 6 to 12 carbon atoms can be phenoxy.

The arylalkyloxy group comprising an alkyl moiety having 1 to 8 carbon atoms can be benzyloxy.

The alkoxycarbonyl group having 2 to 9 carbon atoms can be ethoxycarbonyl.

The alkylcarbamoyl group having 2 to 9 carbon atoms can be methylcarbamoyl.

The alkylsulfamoyl group having 1 to 8 carbon atoms can be methylsulfamoyl, ethylsulfamoyl, propylsulfamoyl, or isopropylsulfamoyl.

The dialkylsulfamoyl group having 2 to 16 carbon atoms can be dimethylsulfamoyl.

The acylamino group having 2 to 8 carbon atoms can be acetylamino.

The alkoxy group having 1 to 8 carbon atoms can be methoxy, ethoxy, propoxy, isopropoxy, butoxy, isobutoxy, t-butoxy, pentyloxy, or hexyloxy.

The haloalkoxy group having 1 to 8 carbon atoms and 1 to 3 halogen atoms can be methoxy, ethoxy, propoxy, isopropoxy, butoxy, or t-butoxy substituted with one to three halogen atoms such as a fluoro atom, a chloro atom, or a bromo atom, and preferably is trifluoromethoxy, chloromethoxy, 2-chloroethoxy, 2-bromoethoxy, or 2-fluoroethoxy.

The pharmacologically acceptable salt of the compound represented by the formula (I) can be an oxalate salt or a hydrochloride salt.

The compound of the present invention can be geometrical isomer or optical isomer such as optically active substance and racemic modification, each of which is included within the scope of the invention.

The following schemes for synthesis relate to the compound represented by the formula (I) wherein R¹=R²=R³=H, and n=m=1.

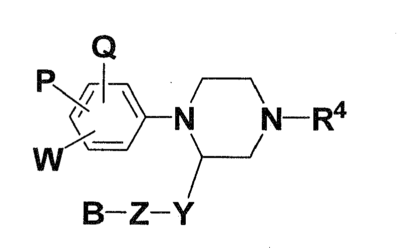

### (1) Compound having Y-Z represented by CH₂O

The compound (h) having Y-Z represented by CH₂O is prepared according to the following method A.

### (Method A)

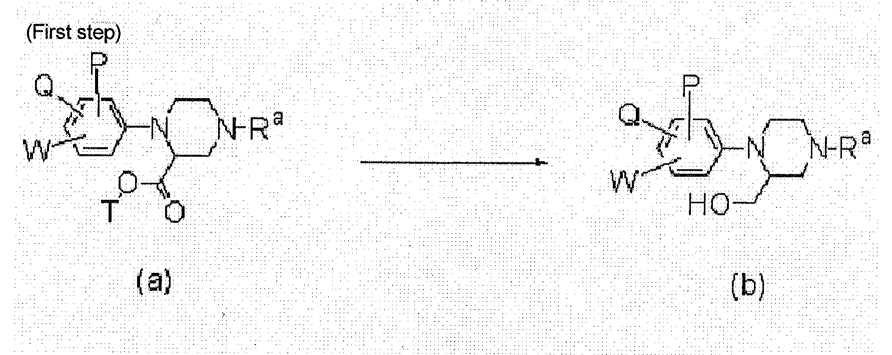

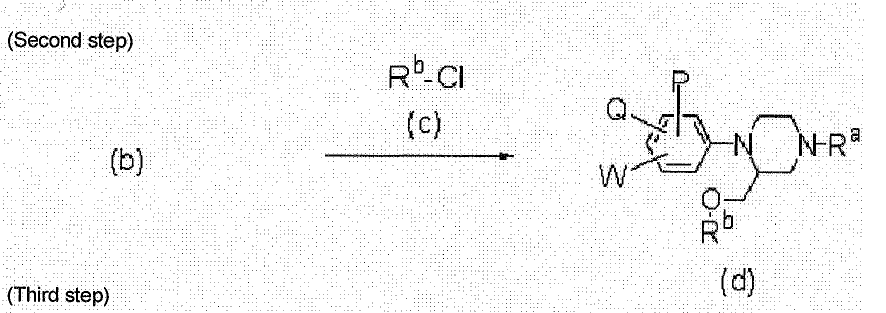

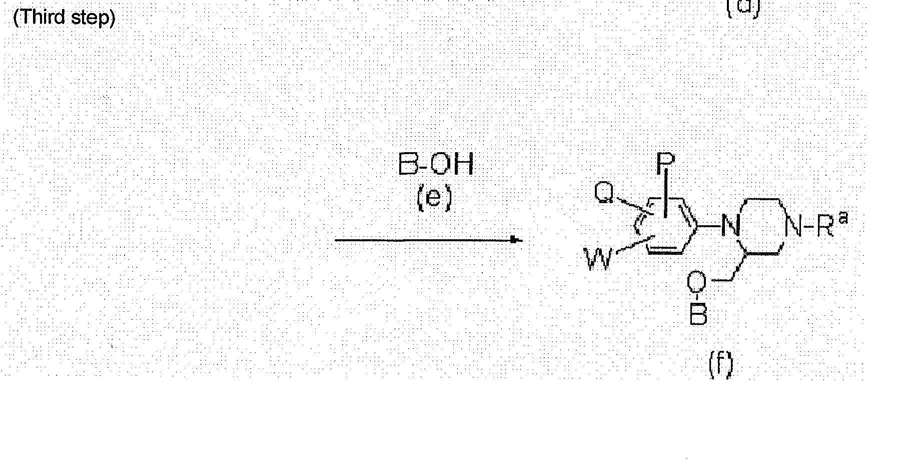

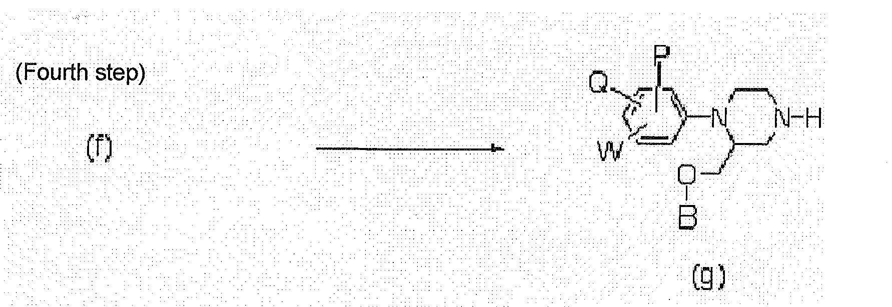

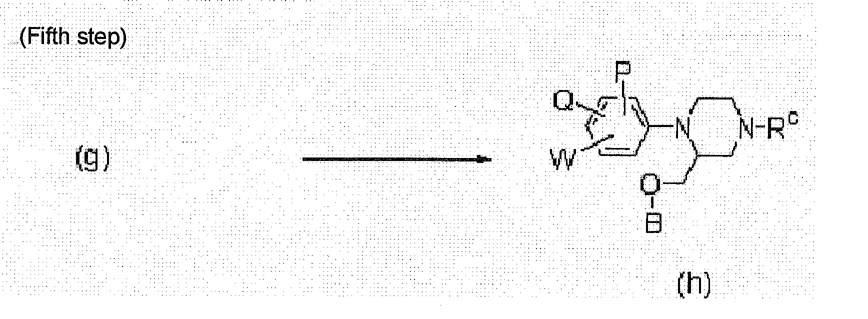

In the formulas, T represents an alkyl group having 1 to 6 carbon atoms, R^{a} represents a protective group such as benzyl, tert-butoxycarbonyl, benzyloxycarbonyl, R^{b} represents methanesulfonyl, p-toluenesulfonyl, benzenesulfonyl or the like, R^{c} represents R⁴ except a hydrogen atom, and P, Q, W and B are the same as those described above.
1) The starting material (a) is synthesized according to a known method (such as E. Woo et al., J. Org. Chem., 1992, 57, 6257; Corsico N. et al., Farmaco, Ed., Sci., 1984, 39, 450; and WO 2003/015784) or an analogous method thereof.
2) The first step
   The reduction of the starting material (a) is conducted using a reducing agent such as lithium aluminum hydride, sodium bis(2-methoxyethoxy)aluminum hydride, diisobutyl aluminum hydride in an inert solvent such as toluene, tetrahydrofuran, benzene, xylene. The reaction temperature is in the range of 0°C to 80°C.
3) The second step
   The compound of the formula (b) is converted into the compound of the formula (d) conducting a reaction with the sulfonyl chloride represented by the formula (c) in the presence of a base such as pyridine, triethylamine, in an inert solvent such as toluene, dichloromethane. Pyridine can be a solvent as well as a base.
4) The third step
   The compound of the formula (d) is converted into the compound of the formula (f) conducting a reaction with the phenol or heteroaryl alcohol represented by the formula (e) in the presence of a base such as sodium hydride, potassium carbonate in an inert solvent such as N,N-dimethylformamide, acetone. The reaction temperature is in the range of 20°C to 80°C.
5) The fourth step
   The protective group (Ra) is eliminated from the amino group in the compound of the formula (f) according to a conventional process. For example, the process can be conducted using 1-chloroethyl chloroformate in an inert solvent such as 1,2-dichloroethane in the case that the protective group is benzyl. The process can also be conducted by catalytic hydrogenation using a catalyst such as palladium-carbon in an inert solvent such as methanol, ethanol.
6) The fifth step
   The compound of the formula (g) is converted into the compound of the formula (h) producing an imine with a ketone or an aldehyde in an inert solvent such as 1,2-dichloroethane, tetrahydrofuran, acetonitrile, and then reducing the imine with sodium triacetoxyborohydride or sodium cyanoborohydride.

The compound (f), which is the intermediate as well as the compound of the present invention, can also be prepared according to the following method B.

### (Method B)

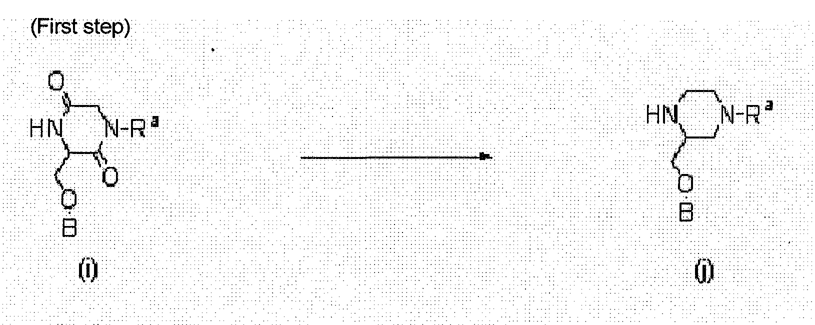

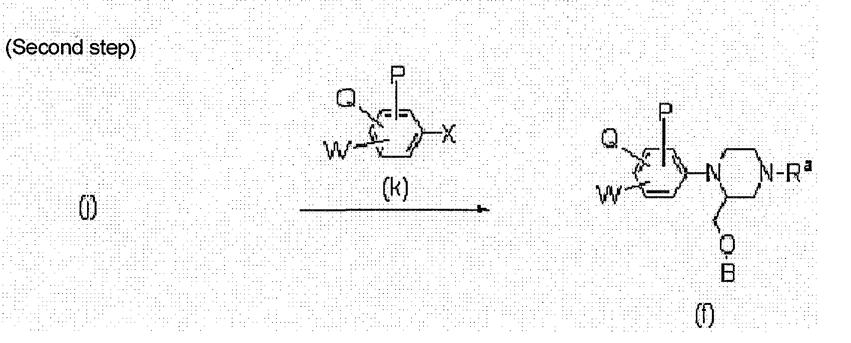

In the formulas, X represents a chloro atom, a bromo atom, an iodo atom, and R^{a}, P, Q, W, and B are the same as those described above.
1) The starting material (i) is synthesized according to a known method (such as WO 2004/089915) or an analogous method thereof.
2) The first step
   The compound of the formula (i) is converted into the compound of the formula (j) using a reducing agent such as lithium aluminum hydride, sodium bis(2-methoxyethoxy)aluminum hydride, diborane in an inert solvent such as tetrahydrofuran, toluene, benzene.
3) The second step

The compound of the formula (j) is converted into the compound of the formula (f) by a reaction with the aryl halide represented by the formula (k) in the presence of a base such as sodium tert-butoxide, tripotassium phosphate, lithium hexamethyldisilazide and a catalyst such as bis(tri-tert-butylphosphine)palladium in an inert solvent such as toluene, tetrahydrofuran. The reaction temperature is in the range of 60°C to 100°C.

In place of bis(tri-tert-butylphosphine)palladium, a palladium catalyst such as bis(dibenzylideneacetone)palladium(0), palladium(II) acetate can be used in combination with a ligand such as 2-dicyclohexylphosphino-2',4',6'-triisopropylbiphenyl, 2-dicyclohexylphosphino-2',6'-dimethoxybiphenyl.

### (2) Compound having Y-Z represented by CH₂S

The compound (p) having Y-Z represented by CH₂S is prepared according to the following method C.

### (Method C)

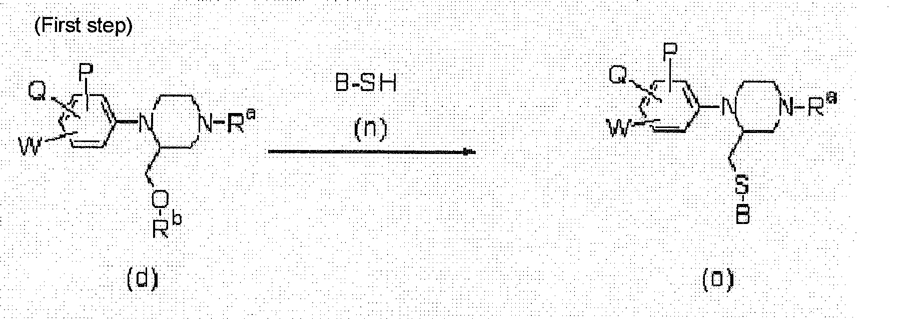

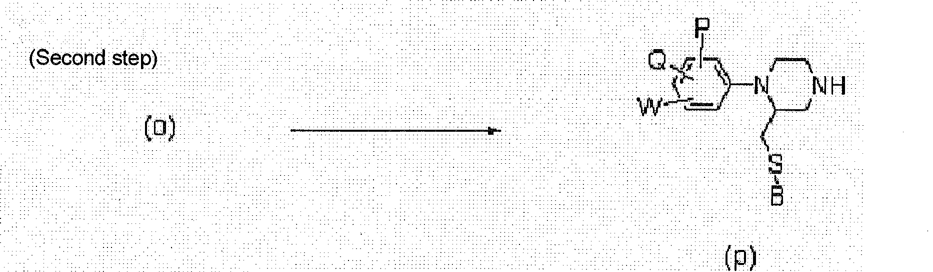

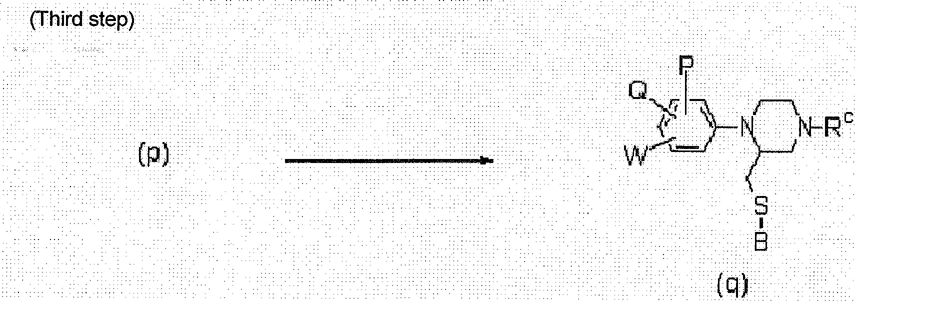

In the formulas, R^{a}, R^{b}, R^{c}, P, Q, W, and B are the same as those described above.
1) The starting material (d) is synthesized in the same manner as in the method A.
2) The first step
   The compound of the formula (d) is converted into the compound of the formula (o) by a reaction with the thiol represented by the formula (n) in the presence of a base such as cesium carbonate, potassium carbonate, sodium hydride in an inert solvent such as acetonitrile, tetrahydrofuran.
3) The second step
   The protective group (Ra) is eliminated from the amino group of the compound of the formula (o) in the same manner as in the process of the method A.
4) The third step

The compound of the formula (p) is converted into the compound of the formula (q) in the same manner as in the process of the method A.

### (2) Compound having Y-Z represented by CH₂NR⁵

The compound (v) having Y-Z represented by CH₂NR⁵ is prepared according to the following method D.

### (Method D)

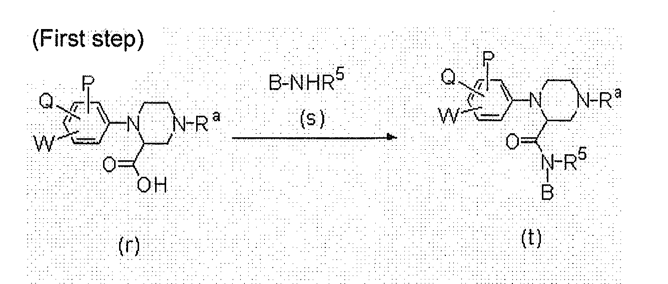

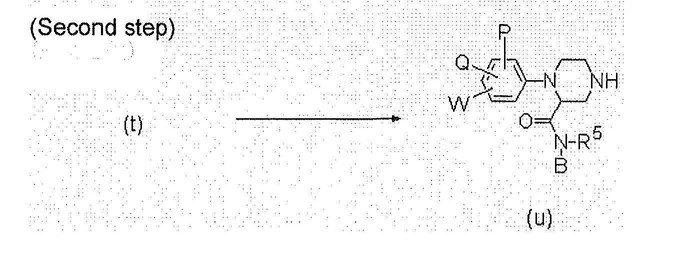

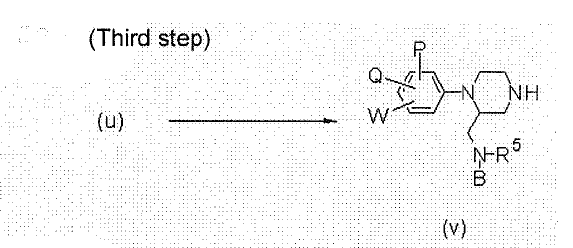

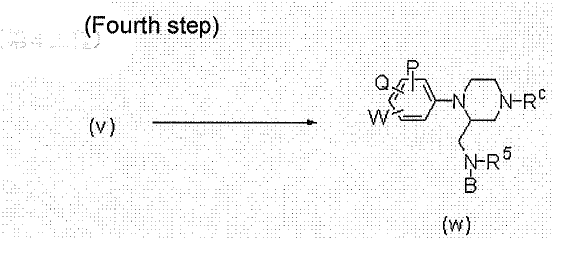

In the formulas, R^{a}, R^{c}, R⁵, P, Q, W, and B are the same as those described above.
1) The starting material (r) is synthesized according to a known method (such as WO 2003/015784) or an analogous method thereof.
2) The first step
   The starting material (r) is converted into the compound of (t) by condensation with the amine represented by the formula (s) in the presence of a base such as N-methylmorpholine, triethylamine, a condensing agent such as 1-ethyl-3-(3-dimethylaminopropyl)carbodiimide hydrochloride (WSC·HCl), and 1-hydroxybenzotriazole in an inert solvent such as N,N-dimethylformamide, dichloromethane. The reaction temperature is in the range of 0°C to 20°C.
3) The second step
   The protective group (Ra) is eliminated form the amino group in the compound of the formula (t) according to a conventional process. For example, the process can be conducted using 1-chloroethyl chloroformate in an inert solvent such as 1,2-dichloroethane in the case that the protective group is benzyl. The process can also be conducted by catalytic hydrogenation using a catalyst such as palladium-carbon in an inert solvent such as methanol, ethanol.
4) The third step
   The compound of the formula (u) is converted into the compound of the formula (v) using a reducing agent such as lithium aluminum hydride, sodium bis(2-methoxyethoxy)aluminum hydride, diborane in an inert solvent such as tetrahydrofuran, toluene.
5) The fourth step

The compound of the formula (v) is converted into the compound of the formula (v) in the same manner as in the process of the method A.

The compound represented by the formula (I) or its pharmacologically acceptable salt can also be prepared by referring to the below described Examples and the prior art documents.

Examples of the obtained compounds of the present invention are shown below.

### (Compound type 1)

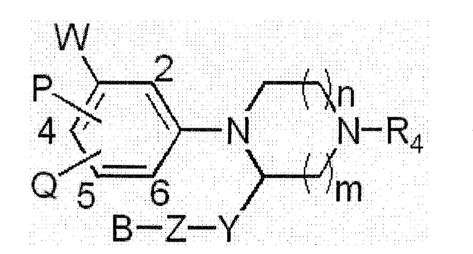

In the formulas, B, Z, Y, W, P, Q, R⁴, m, and n are described in the following Tables 1 to 3.

**TABLE 1**

| B | Z | Y | W | P | Q | R⁴ | m | n |
|---|---|---|---|---|---|---|---|---|
| 4-chlorophenyl | O | CH₂ | iPr | H | H | H | 1 | 1 |
| 4-t-butoxyphenyl | O | CH₂ | iPr | H | H | H | 1 | 1 |
| 4-cyanophenyl | O | CH₂ | iPr | H | H | H | 1 | 1 |
| 4-sulfamoylphenyl | O | CH₂ | iPr | H | H | H | 1 | 1 |
| 4-isopropylsulfamoylphenyl | O | CH₂ | iPr | H | H | H | 1 | 1 |
| 4-fluorophenyl | O | CH₂CH₂ | Et | H | 5-OMe | H | 1 | 1 |
| 4-aminophenyl | S | CH₂ | iPr | 4-OH | 5-OH | Me | 1 | 1 |
| 4-dimethylaminophenyl | O | CH₂ | iPr | 4-F | H | H | 1 | 1 |
| 4-phenoxyphenyl | O | CH₂ | Me | 4-NMe₂ | H | H | 1 | 1 |
| 4-phenoxyphenyl | S | CH₂ | iPr | 4-NO₂ | H | H | 1 | 1 |

**TABLE 2**

| B | Z | Y | W | P | Q | R⁴ | m | n |
|---|---|---|---|---|---|---|---|---|
| 3,4-methylenedioxyphenyl | O | CH₂ | tBu | 4-CF₃ | H | H | 1 | 1 |
| 4-hydroxyphenyl | O | CH₂ | Pr | 4-F | H | H | 1 | 1 |
| 4-benzyloxyphenyl | O | CH₂ | secBu | H | H | H | 1 | 1 |
| 4-trifluoromethylphenyl | O | CH₂ | iBu | H | H | H | 1 | 1 |
| 1-naphthyl | S | CH₂ | iPr | 2- OMe | H | Me | 1 | 1 |
| 2-naphthyl | O | CH₂ | iPr | H | H | Et | 1 | 1 |
| benzofuran-6-yl | NH | CH₂ | cyclopropyl | H | H | H | 2 | 1 |
| 6-indolyl | O | CH₂ | cyclohexyl | H | H | H | 1 | 2 |
| 2-thienyl | O | CH₂ | secBu | H | H | H | 1 | 1 |
| 3,4-dimethoxyphenyl | NH | CH₂ | secBu | 2-OMe | 6-OMe | H | 1 | 1 |
| 3,4-dihydroxyphenyl | NMe | CH₂ | tBu | 2-F | H | H | 1 | 1 |
| 4-Ssulfamoylphenyl | S | H₂ | iPr | H | H | H | 2 | 1 |
| 4-t-butylsulfamoylphenyl | O | CH₂ | iPr | 4-Cl | H | H | 1 | 1 |
| 4-trifluoromethoxyphenyl | O | CH₂ | Et | H | 5-OMe | H | 1 | 1 |
| 4-acetylaminophenyl | S | CH₂ | iPr | 4-OH | 5-OH | H | 1 | 1 |

**TABLE 3**

| B | Z | Y | W | P | Q | R⁴ | m | n |
|---|---|---|---|---|---|---|---|---|
| 4-dimethylaminophenyl | O | CH₂ | iPr | 4-F | H | H | 1 | 1 |
| 3-phenoxyphenyl | O | CH₂ | Me | 4-NMe2 | H | H | 1 | 1 |
| 3-phenoxyphenyl | S | CH₂ | iPr | 4- NO₂ | H | H | 1 | 1 |
| 3-sulfamoylphenyl | O | CH₂ | tBu | 4-CF₃ | H | H | 1 | 1 |
| 3-isopropylsulfamoylphenyl | O | CH₂ | Pr | 4-F | H | H | 1 | 1 |
| 4-benzyloxyphenyl | O | CH₂CH₂ | secBu | H | H | H | 1 | 1 |
| 4-trifluoromethylphenyl | O | CH₂CH₂ | iBu | H | H | H | 1 | 1 |
| 1-naphthyl | S | CH₂CH₂ | iPr | H | H | H | 1 | 1 |
| 2-naphthyl | O | CH₂CH₂ | iPr | H | H | Et | 1 | 1 |
| benzofuran-6-yl | NH | CH2 | cyclo-butyl | H | H | H | 2 | 1 |
| 6-indolyl | NH | CH₂ | cyclo-propyl | H | H | H | 1 | 2 |
| 2-thienyl | S | CH₂CH₂ | secBu | H | H | H | 1 | 1 |
| 4-chlorophenyl | S | CH₂ | secBu | 2-OMe | 6- | H | 1 | 1 |
| 4-fluorophenyl | NMe | CH₂ | tBu | 4-OMe | H | H | 1 | 1 |

### (Compound type 2)

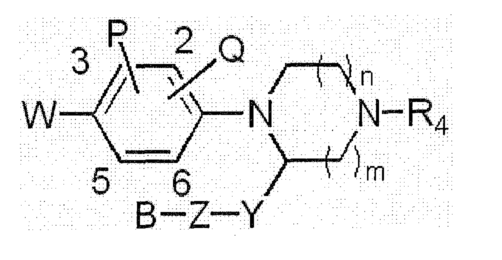

In the formulas, B, Z, Y, W, P, Q, and R⁴ are described in the following Tables 4 to 6.

**TABLE 4**

| B | Z | Y | W | P | Q | R⁴ | m | n |
|---|---|---|---|---|---|---|---|---|
| 3,4-methylenedioxyphenyl | O | CH₂ | iPr | H | H | H | 1 | 1 |
| 4-hydroxyphenyl | O | CH₂ | iPr | H | H | H | 1 | 1 |
| 4-benzyloxyphenyl | O | CH₂ | iPr | H | H | H | 1 | 1 |
| 4-sulfamoy-phenyl | O | CH₂ | iPr | H | H | H | 1 | 1 |
| 4-isopropyl-sulfamoylphenyl | O | CH₂ | iPr | H | H | H | 1 | 1 |
| 4-chlorophenyl | O | CH₂ | Pr | H | H | H | 1 | 1 |
| 4-chlorophenyl | O | CH₂ | tBu | H | H | H | 1 | 1 |
| 4-chlorophenyl | O | CH₂ | cyclohexyl | H | H | H | 1 | 1 |

**TABLE 5**

| B | Z | Y | W | P | Q | R⁴ | m | n |
|---|---|---|---|---|---|---|---|---|
| 4-chlorophenyl | O | CH₂ | (CH₂)₇CH₃ | H | H | H | 1 | 1 |
| 4-chlorophenyl | O | CH₂ | iPr | H | H | H | 1 | 1 |
| 3-isopropylsulfamoylphenyl | O | CH₂ | iPr | H | H | H | 1 | 1 |
| phenoxyphenyl | O | CH₂ | iPr | H | H | H | 1 | 1 |
| phenoxyphenyl | S | CH₂ | iPr | H | H | H | 1 | 1 |
| 4-trifluoromethylphenyl | O | CH₂ | tBu | 2-CF₃ | H | H | 1 | 1 |
| 4-hydroxyphenyl | O | CH₂ | Pr | F | F | H | 2 | 1 |
| 4-benzyloxyphenyl | O | CH₂ | secBu | H | H | H | 1 | 1 |
| benzofuran-6-yl | O | CH₂ | iBu | F | F | H | 1 | 1 |
| 1-naphthyl | O | CH₂ | iPr | H | H | H | 2 | 1 |
| 2-naphthyl | O | CH₂ | iPr | 3-OMe | H | Et | 1 | 1 |

**TABLE 6**

| B | Z | Y | W | P | Q | R⁴ | m | n |
|---|---|---|---|---|---|---|---|---|
| benzofuran-6-yl | O | CH₂ | iBu | H | H | H | 1 | 1 |
| 6-indolyl | O | CH₂ | iBu | H | H | H | 1 | 1 |
| 2-thienyl | O | CH₂ | secBu | H | H | H | 1 | 1 |
| 3,4-dimethoxyphenyl | O | CH₂ | Me | H | H | H | 1 | 1 |
| 3,4-dihydroxy phenyl | NEt | CH₂ | iPr | H | H | H | 1 | 1 |
| 4-benzyloxyphenyl | S | CH₂ | Et | H | H | Me | 1 | 1 |
| 4-sulfamoy-phenyl | NH | CH₂ | iPr | H | H | Me | 2 | 1 |
| 4-isopropylsulfamoylphenyl | NMe | CH₂ | iPr | H | H | H | 1 | 1 |
| 4-fluorophenyl | S | CH₂ | Pr | 3-OMe | 5-OMe | H | 1 | 1 |
| 4-trifluoromethoxyphenyl | O | CH₂ | tBu | 3-OMe | 5-OMe | H | 2 | 1 |
| 4-chlorophenyl | S | CH₂ | cyclo-propyl | H | H | H | 1 | 1 |
| 4-chlorophenyl | O | CH₂ | (CH2)7CH₃ | H | H | H | 1 | 1 |
| 4-chlorophenyl | S | CH₂ | cyclo-butyl | H | H | H | 1 | 1 |
| 3-t-butylsulfamoylphenyl | O | CH₂ | iPr | 3-OMe | 5-OMe | H | 2 | 1 |
| 4-phenoxyphenyl | O | CH₂ | iPr | H | H | H | 1 | 1 |
| 4-phenoxyphenyl | S | CH₂ | iPr | H | H | H | 1 | 1 |

### (Compound type 3)

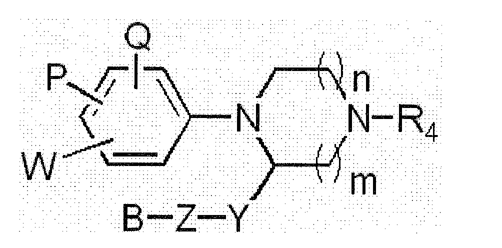

In the formulas, B, Z, Y, W, P, Q, R⁴, m, and n are described in the following Tables 7 and 8.

**TABLE 7**

| B | Z | Y | W | P | Q | R⁴ | m | n |
|---|---|---|---|---|---|---|---|---|
| 4-chlorophenyl | O | CH₂ | 3,5-tBu | H | H | H | 1 | 1 |
| 4-chlorophenyl | O | CH₂ | 2,6-Me | H | H | H | 1 | 1 |
| 4-chlorophenyl | O | CH₂ | 2,4,6-Me | H | H | H | 1 | 1 |
| 3,4-methylenedioxyphenyl | O | CH₂ | 3,5-Me | 4-OMe | H | H | 2 | 1 |
| 4-hydroxyphenyl | O | CH₂ | 3,5-Pr | 4-OH | H | H | 1 | 1 |
| 4-benzyloxyphenyl | O | CH₂ | 3,5-tBu | H | H | H | 1 | 2 |
| 4-sulfamoylphenyl | O | CH₂ | 3,4,5-Me | H | H | H | 1 | 1 |
| 4-isopropylsulfamoylphenyl | O | CH₂ | 3,4,5-Me | H | H | H | 1 | 1 |
| 4-chlorophenyl | O | CH₂ | 3,4,5-Me | H | H | H | 1 | 1 |
| 4-chlorophenyl | O | CH₂ | 3,5-Pr | H | H | H | 2 | 1 |

**TABLE 8**

| B | Z | Y | W | P | Q | R⁴ | m | n |
|---|---|---|---|---|---|---|---|---|
| 4-chloropyridyl | O | CH₂ | 3,5-cyclopropyl | H | H | H | 1 | 1 |
| 4-chlorophenyl | O | CH₂CH₂ | 3,5-tBu | H | H | H | 1 | 1 |
| 4-chlorophenyl | O | CH₂ | 3,5-iPr | H | H | H | 2 | 1 |
| 3-isopropylsulfamoylphenyl | NMe | CH₂CH₂ | 3,5-tBu | H | H | Me | 1 | 1 |
| 4-phenoxyphenyl | NH | CH₂ | 2,4,6-Me | H | H | H | 1 | 1 |
| 4-phenoxyphenyl | S | CH₂ | 3,4-iPr | H | H | H | 1 | 1 |
| 4-trifluoromethylphenyl | O | CH₂ | 3,5-Me | H | H | H | 1 | 1 |
| 4-hydroxyphenyl | O | CH₂ | 3,5-Pr | H | H | H | 2 | 1 |
| 4-benzyloxyphenyl | O | CH₂ | 3,5-tBu | H | H | H | 1 | 1 |
| benzofuran-6-yl | O | CH₂CH₂ | 3,4,5-Me | H | H | Et | 1 | 1 |
| 1-naphthyl | S | CH₂ | 3,4,5-Me | H | H | H | 1 | 1 |
| 2-naphthyl | O | CH₂ | 3,4,5-Me | H | H | H | 1 | 1 |
| benzofuran-6-yl | O | CH₂CH₂ | 3,5-Pr | H | H | H | 1 | 1 |
| 6-indolyl | O | CH₂ | 3,5-cyclohexyl | H | H | H | 1 | 1 |
| 2-thienyl | O | CH₂ | 3,5-tBu | H | H | H | 1 | 1 |

### (Compound type 4)

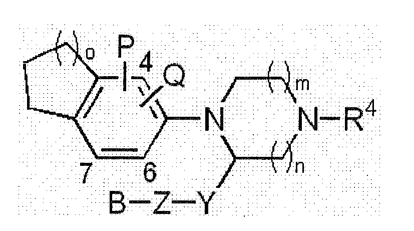

In the formulas, B, Z, Y, P, Q, R⁴, m, n, and o are described in the following Tables 9 and 10.

**TABLE 9**

| B | Z | Y | P | Q | R⁴ | m | n | o |
|---|---|---|---|---|---|---|---|---|
| 4-chlorophenyl | O | CH₂ | H | H | H | 1 | 1 | 1 |
| 4-hydroxyphenyl | O | CH₂ | H | H | H | 1 | 1 | 1 |
| 4-benzyloxyphenyl | O | CH₂CH₂ | 4-F | H | H | 1 | 1 | 1 |
| 4-sulfamoylphenyl | O | CH₂ | 6-Cl | H | H | 1 | 1 | 1 |
| 4-isopropylsulfamoylphenyl | O | CH₂ | 4-OMe | H | H | 1 | 1 | 1 |
| 3, 4-methylenedioxyphenyl | O | CH₂ | 4-CF₃ | H | H | 1 | 2 | 2 |
| 4-fluorophenyl | S | CH₂ | H | H | H | 1 | 1 | 1 |
| 4-chlorophenyl | NH | CH₂CH₂ | H | H | H | 1 | 1 | 1 |
| 4-methylphenyl | NMe | CH₂ | H | H | H | 1 | 1 | 2 |

**TABLE 10**

| B | Z | Y | P | Q | R⁴ | m | n | o |
|---|---|---|---|---|---|---|---|---|
| 4-chlorophenyl | NH | CH₂CH₂ | H | H | H | 2 | 1 | 2 |
| 3-isopropylsulfamoylphenyl | O | CH₂ | H | H | H | 1 | 1 | 1 |
| 4-phenoxyphenyl | O | CH₂ | H | H | H | 1 | 1 | 1 |
| 4-phenoxyphenyl | S | CH₂CH₂ | H | H | H | 2 | 1 | 1 |
| 4-trifluoromethylphenyl | O | CH₂ | 4-OMe | H | H | 1 | 1 | 1 |
| 4-hydroxyphenyl | NH | CH₂ | 4-OH | H | H | 1 | 1 | 1 |
| 4-benzyloxyphenyl | O | CH₂ | 6-OMe | H | H | 1 | 1 | 1 |
| benzofuran-6-yl | O | CH₂ | H | H | H | 1 | 1 | 2 |
| 1-naphthyl | S | CH₂ | F | F | Me | 2 | 1 | 1 |
| 2-naphthyl | O | CH₂ | H | H | Et | 1 | 1 | 1 |
| benzofuran-6-yl | O | CH₂ | H | H | H | 1 | 1 | 2 |
| 6-indolyl | O | CH₂ | H | H | H | 1 | 1 | 1 |
| 2-thienyl | O | CH₂ | H | H | H | 1 | 1 | 1 |

The pharmacological tests of the invention are described below.

### 1. P2X₄ antagonism study by measurement of intracellular Ca²⁺ influx response

1321N1 cells stably expressing human P2X₄ receptors were adopted for calcium influx assay. P2X4/1321N1 cells were plated in 96-well assay plate and cultured 24 hours in an atmosphere of 5 % CO₂ at 37°C. Fura-2 AM calcium indicator in assay buffer (150 mM NaCl, 5 mM KCl, 1.8 mM CaCl₂, 1.2 mM MgCl₂, 10 mM D-glucose and 25 mM HEPES, pH 7.4) was loaded onto cells for 1 hour at room temperature and the fluorescence was detected by FLUOstar Optima micro plate reader (BMG labtech). The cells were alternatively illuminated with two excitations wavelengths (340 nm and 380 nm) via xenon lamp and the emitted fluorescence was measured at 510 nm. The fluorescence changes after the treatment of 1 µM ATP were monitored and determined the fluorescence ratio (F₃₄₀/F₃₈₀) as the index of intracellular calcium change. Tested compound were treated to cells 15 min before the addition of ATP and the inhibition activity of compounds was calculated by comparing the Ca²⁺ response with control in the absence of tested compound.

### 2. [³H]paroxetine binding to Serotonin transporter

To investigate the effects of compounds on human Serotonin transporter, [³H]paroxetine competition binding study was performed. Cell membranes prepared from HEK-293 cells stably expressing the human Serotonin transporter (1 mg protein/ml) were incubated with 0.4 nM [³H]paroxetine in the presence of tested compound in incubation buffer containing 50 mM Tris-HCl, pH7.4, 120 mM NaCl and 5 mM KCl. After incubation for 60 min at 25°C, the reaction was terminated by rapid filtration through GF/B glass fiber filter and rinsed with ice-cold incubation buffer. The retained radioactivity on filter was measured by a liquid scintillation counter. Nonspecific binding was defined with 10 µM imipramine and subtracted from total binding to determine specific binding. Inhibition activities of tested compounds were determined as a percent inhibition of the specific binding.

### 3. Serotonin reuptake inhibition assay

To test the serotonin reuptake inhibitory activity of compounds, experiments were carried out as described by Provic and Muller (Arzneim-Forsch. Drug Res., 1995, 45:1145-1148.). 100 µg of the synaptosomes prepared from the rat brain were incubated for 15 min at 37°C with 0.1 µCi [³H]serotonin in the presence of tested compound in a buffer containing 106.2 mM NaCl, 4.5 mM KCl, 2.25 mM MgSO₄, 1.08 mM NaH₂PO₄, 22.5 mM NaHCO₃, 9.9 mM glucose, 9 µM EGTA and 45 µM ascorbic acid (pH 7.4). Basal control activity was determined by incubating the same mixture for 15 min at 4°C in the presence of 10 µM imipramine. Following incubation, the samples were filtered rapidly under vacuum through GF/B glass fiber filters and rinsed twice with ice-cold incubation buffer using a cell harvester. The retained radioactivity on filters was measured in TopCount scintillation counter. The results were expressed as a percent inhibition of the specific binding in the absence of tested compound.

As is evident from the below-described results shown in Table 11 of Example 19, the compound represented by the formula (I) or its pharmacologically acceptable salt shows excellent P2X₄ receptor antagonism.

As is also evident from the results shown in Tables 12 and 13, the compound represented by the formula (I) shows weak binding affinity to serotonin transporter.

Therefore, it is considered that the compound represented by the formula (I) or its pharmacologically acceptable salt, which shows P2X₄ receptor antagonism, are effective as an agent for prevention and treatment of neuropathic pains. The compound, which shows weak binding affinity to serotonin transporter, is also expected to show side effect weaker than that of SSRI.

The compound represented by the formula (I) or its pharmacologically acceptable salt is effective to prevent or treat pains caused viral diseases such as herpes.

If desired, the compound represented by the formula (I) or its pharmacologically acceptable salt can be employed in combination with other medical agents such as opioide analgesics (morphine, fentanyl), sodium channel blockers (novocaine, lidocaine), NSAIDs (aspirin, ibuprofen).

The compound represented by the formula (I) or its pharmacologically acceptable salt can be administered to human beings by ordinary administration methods such as oral administration or parenteral administration.

The compound can be granulated in ordinary manners for the preparation of pharmaceuticals. For instance, the compound can be processed to give pellets, granule, powder, capsule, suspension, injection, suppository, and the like.

For the preparation of these pharmaceuticals, ordinary additives such as vehicles, disintegrators, binders, lubricants, dyes, and diluents. As the vehicles, lactose, D-mannitol, crystalline cellulose, and glucose can be mentioned. Further, there can be mentioned starch and carboxymethylcellulose calcium (CMC-Ca) as the disintegrators, magnesium stearate and talc as the lubricants, and hydroxypropylcellulose (HPC), gelatin and polyvinylpirrolidone (PVP) as the binders. The preparation of an injection can be made using solvents, stabilizers, dissolution-aids, suspensions, emulsifiers, soothing agents, buffers, preservatives, and the like.

The compound of the invention can be administered to an adult generally in an amount of approx. 0.01 mg to 100 mg a day by parenteral administration and 1 mg to 2,000 mg a day by oral administration. The dosage can be adjusted in consideration of age and conditions of the patient.

The present invention is further described by the following non-limiting examples.

### Examples

### Example 1

### 2-(4-Chlorophenoxymethyl)-1-(4-isopropylphenyl)piperazine hydrochloride

### (1) N-Benzyl-N'-(4-isopropylphenyl)ethane-1,2-diamine hydrochloride

A suspension of 3-benzyloxazolidin-2-one (2.00 g, 11.29 mmol) and 4-isopropylaniline hydrochloride (1.94 g, 11.29 mmol) in 2-(2-methoxyethoxy)ethanol (2 mL) was heated at 170°C for 12 hours. After cooling, ethyl acetate (10 mL) was added, and the mixture was refluxed for 2 hours. After the mixture was cooled to room temperature, the precipitate was collected, and washed with small amounts of ethyl acetate. The resultant crude product was recrystallized from ethanol to give the titled compound (1.31 g, 38%).
¹H NMR (CDCl₃, 400 MHz) 5: 1.15 (6 H, d, J = 7 Hz), 2.7-2.8 (1 H, m), 3.00 (2 H, t, J = 5 Hz), 3.48 (2 H, t, J = 5 Hz), 3.97 (2 H, s), 6.58 (2 H, d, J = 9 Hz), 6.97 (2 H, d, J = 8 Hz), 7.3-7.4 (3 H, m), 7.4-7.5 (2 H, m).

### (2) Ethyl 4-benzyl-1-(4-isopropylphenyl)piperazine-2-carboxylate

A saturated aqueous sodium hydrogen carbonate solution (5 mL) was added to a solution of N-benzyl-N'-(4-isopropylphenyl)ethane-1,2-diamine hydrochloride (650 mg, 2.13 mmol) in chloroform (5 mL), and the mixture was stirred for 30 minutes. The organic layer was collected, and the aqueous layer was extracted three times with chloroform. The combined organic layers were dried over anhydrous sodium sulfate, and the solvent was removed under reduced pressure. The residue and triethylamine (554 µL, 2.13 mmol) was dissolved in dry benzene (2 mL), and the resulting solution was added dropwise over 30 minutes at 40°C to a solution of ethyl 2,3-dibromopropionate (1.16 g, 4.46 mmol) in benzene (2 mL). After refluxing for 15 hours, the reaction mixture was poured into a saturated aqueous sodium hydrogen carbonate solution, and was extracted with ethyl acetate. The organic extracts were washed with saturated brine, dried over anhydrous sodium sulfate, and the solvent was removed under reduced pressure. The residue was purified by silica gel column chromatography (hexane/ethyl acetate =20/1), to give the titled compound (323 mg, yield 41%).
¹H NMR (CDCl₃, 400 MHz) 5: 1.12 (3 H, t, J = 7 Hz), 1.20 (6 H, d, J = 7 Hz), 2.34 4 (1 H, dt, J = 4, 11 Hz), 2.44 (1H, dd, J = 4, 11 Hz), 2.7-2.9 (1 H, m), 2.94 (1 H, d, J = 9 Hz), 3.28 (1 H, dt, J = 2, 11 Hz), 3.38 (1 H, dt, J = 3, 11 Hz), 3.43 (1 H, d, J = 13 Hz), 3.59 (1 H, dt, J = 3, 11 Hz), 3.63 (1 H, d, J = 13 Hz), 4.0-4.2 (2 H, m), 4.37 (1 H, t, J = 3 Hz), 6.78 (2 H, d, J = 9 Hz), 7.09 (2H, d, J = 9 Hz), 7.2-7.3 (5 H, m).

### (3) [4-Benzyl-1-(4-isopropylphenyl)piperazin-2-yl]methanol

To an ice-cold suspension of lithium aluminum hydride (80 mg, 2.10 mmol) in THF (4 mL) was added, dropwise over 15 minutes, a solution of ethyl 4-benzyl-1-(4-isopropylphenyl)piperazine-2-carboxylate (700 mg, 1.90 mmol) in toluene (8 mL), and the mixture was stirred at room temperature for 2 hours. The reaction mixture was treated with saturated aqueous sodium sulfate solution, and resulting insoluble materials were filtered off. The filtrate was dried over anhydrous sodium sulfate, and the solvent was removed under reduced pressure to give the titled compound (578 mg, yield 94%).
¹H NMR (CDCl3, 400 MHz) δ: 1.21 (6 H, d, J = 7 Hz), 2.35 (1 H, dt, J = 4, 11 Hz), 2.61 (1 H, dd, J = 3, 11 Hz), 2.7-2.9 (1 H, m), 2.93 (1 H, d, J = 10 Hz), 3.09 (1 H, d, J = 11 Hz), 3.32 (1 H, dt, J = 3, 11 Hz), 3.51 (1 H, dt, J = 3, 11 Hz), 3.54 (2 H, s), 3.7-3.8 (2 H, m), 3.92 (1 H, d, J = 9 Hz), 4.20 (1 H, br s), 6.83 (2 H, d, J = 9 Hz), 7.11 (2 H, d, J = 9 Hz), 7.1-7.4 (5 H, m) .

### (4) 4-Benzyl-1-(4-isopropylphenyl)-2-methanesulfonyloxymethylpiperazine

To a solution of [4-benzyl-1-(4-isopropylphenyl)piperazin-2-yl]methanol (578 mg, 1.78 mmol)in pyridine (15 mL) was added methanesulfonyl chloride (173 µL, 2.23 mmol) under ice-cooling, and then the mixture was stirred at room temperature for 16 hours. The reaction mixture was poured into water, extracted with ethyl acetate, and the organic layer was washed with saturated brine. After drying over anhydrous sodium sulfate, and the solvent was removed under reduced pressure. The residue was purified by silica gel column chromatography (hexane/ethyl acetate =3/1), to give the titled compound (716 mg, quantitative).
¹H NMR (CDCl₃, 400 MHz) δ: 1.21 (6 H, d, J = 7 Hz), 2.31 (1 H, dt, J = 4, 11 Hz), 2.39 (1 H, dd, J = 3, 11 Hz), 2.68 (3 H, s), 2.7-2.9 (1 H, m), 2.8-2.9 (1 H, m), 2.97 (1 H, dt, J = 2, 11 Hz), 3.13 (1 H, dt, J = 3, 11 Hz), 3.30 (1 H, dt, J = 3, 11 Hz), 3.48 (1 H, d, J = 13 Hz), 3.59 (1 H, d, J = 13 Hz), 4.07 (1 H, dt, J = 3, 7 Hz), 4.23 (1 H, dd, J = 4, 9 Hz), 4.24 (1 H, t, J = 9 Hz), 6.82 (2 H, d, J= 9 Hz), 7.11 (2 H, d, J = 9 Hz), 7.2-7.4 (5 H, m).

### (5) 4-Benzyl-2-(4-chlorophenoxymethyl)-1-(4-isopropylphenyl)-piperazine

To a solution of p-chlorophenol (415 mg, 3.23 mmol) in N,N-dimethylformamide (6 mL) was added 55% sodium hydride (140 mg, 3.23 mmol) under ice-cooling, and then the mixture was stirred for 20 minutes. Then, to the mixture was added a solution of 4-benzyl-1-(4-isopropylphenyl)-2-methanesulfonyloxymethylpiperazine (1.08 g, 2.69 mmol) in N,N-dimethylformamide (9 mL). After stirring at 60°C for 3 hours, the reaction mixture was poured into water, and was extracted with chloroform. The organic layer was washed with saturated brine, dried over anhydrous sodium sulfate, and the solvent was removed under reduced pressure. The residue was purified by silica gel column chromatography (hexane/ethyl acetate =20/1), to give the titled compound (913 mg, yield 78%).
¹H NMR (CDCl₃, 400 MHz) δ: 1.21 (6 H, d, J = 7 Hz), 2.34 (1H, dt, J = 3, 11 Hz), 2.39 (1H, dd, J = 3, 11Hz), 2.7-2.9 (2 H, m), 3.11 (1 H, dt, J = 2, 11Hz), 3.17 (1H, dt, J = 3, 11 Hz), 3.34 (1 H, dt, J = 3, 11 Hz), 3.51 (1 H, d, J = 13 Hz), 3.59 (1 H, d, J = 13 Hz), 3.86 (1 H, dd, J = 4,9 Hz), 4.0-4.1 (1 H, m), 4.36 (1H, t, J = 9 Hz), 6.6-6.7 (2 H, m), 6.85 (2 H, d, J = 9 Hz), 7.1-7.3 (9 H,m).

### (6) 2-(4-Chlorophenoxymethyl)-1-(4-isopropylphenyl)piperazine

To a solution of 4-benzyl-2-(4-chlorophenoxymethyl)-1-(4-isopropylphenyl)piperazine (1.13 g, 2.59 mmol) in 1,2-dichloroethane (10 mL) was added 1-chloroethyl chloroformate (335 µL, 3.11 mmol) under ice-cooling, and then the mixture was refluxed for 3 hours. After evaporation of the solvent under reduced pressure, the residue was dissolved in methanol (10 mL), and the solution was refluxed for 30 minutes. After evaporation of the solvent under reduced pressure, the residue was purified by silica gel column chromatography (chloroform/methanol =98/2), to give the titled compound (895 mg, quantitative).
¹H NMR (CDCl₃, 400 MHz) δ: 1.22 (6 H, d, J = 7 Hz), 2.7-2.9 (1 H, m), 3.0-3.2 (1 H, m), 3.2-3.4 (4 H, m), 3.46 (1 H, dd, J = 4, 13 Hz), 3.85 (1 H, dd, J = 4, 9 Hz), 4.0-4.1 (1 H, m), 4.33 (1 H, t, J = 9 Hz), 6.76 (2 H, dt, J = 3, 9 Hz), 6.91 (2 H, d, J = 9 Hz), 7.1-7.2 (4 H, m).

### (7) 2-(4-Chlorophenoxymethyl)-1-(4-isopropylphenyl)piperazine hydrochloride

To a solution of 2-(4-chlorophenoxymethyl)-1-(4-isopropylphenyl)piperazine (895 mg, 2.59 mmol) in ethyl acetate (5 mL) was added 4 N hydrogen chloride/ethyl acetate (649 µL), and stirred for 30 minutes. The precipitated crystal was collected by filtration to give the titled compound as a white crystal (814 mg, yield 82%).
mp: 174-176°C
¹H NMR (CD₃OD, 400 MHz) 5: 1.20 (6 H, d, J = 7 Hz), 2.7-2.9 (1 H, m), 3.3-3.5 (4 H, m), 3.5-3.6 (2 H, m), 3.9-4.0 (1 H, m), 4.0-4.1 (2 H, m), 6.81 (2 H, d, J = 9 Hz), 7.04 (2 H, d, J = 9 Hz), 7.1-7.3 (4 H, m). IR (Cm⁻¹, KBr): 2958, 2914, 2760, 2688, 2621, 2519, 2451, 1612, 1593, 1518, 1493, 1456, 1383, 1292, 1273, 1240, 1198, 1173, 1140, 1093, 1041, 958, 933, 895, 827, 795, 665, 623, 555, 507.

The target compounds described in the following Example 2-17 were prepared in a procedure similar to that of Example 1.

### Example 2

### 2-(4-Chlorophenoxymethyl)-1-(4-propylphenyl)piperazine hydrochloride

### (1) 4-Benzyl-2-(4-chlorophenoxymethyl)-1-(4-propylphenyl)piperazine

¹H NMR (CDCl₃, 400 MHz) δ: 0.92 (3 H, t, J = 7 Hz), 1.5-1.7 (2 H, m), 2.3-2.5 (2 H, m), 2.50 (2 H, t, J = 7 Hz), 2.89 (1 H, dd, J = 2, 11 Hz), 3.1-3.2 (2 H, m), 3.33 (1 H, dt, J = 2, 11 Hz), 3.51 (1 H, d, J = 13 Hz), 3.60 (1 H, d, J = 13 Hz), 3.86 (1 H, dd, J = 3, 8 Hz), 4.0-4.1 (1 H, m), 4.36 (1 H, t, J = 9 Hz), 6.68 (2 H, d, J = 9 Hz), 6.84 (2 H, d, J = 9 Hz), 7 .06 (2 H, d, J = 9 Hz), 7.17 (2 H, d, J = 9 Hz), 7.2-7.3 (5 H, m).

### (2) 2-(4-Chlorophenoxymethyl)-1-(4-propylphenyl)piperazine

¹H NMR (CDCl₃, 400 MHz) δ: 0.92 (3 H, t, J = 7 Hz), 1.5-1.7 (2 H, m), 2.50 (2 H, t, J = 7 Hz), 2.9-3.0 (1 H, m), 3.0-3.2 (3 H, m), 3.2-3.3 (2 H,m), 3.84 (1 H, dd, J = 3, 8 Hz), 3.9-4.0 (1 H, m), 4.27 (1 H, t, J = 9 Hz), 6.73 (2 H, d, J = 8 Hz), 6.86 (2 H, d, J = 8 Hz), 7.07 (2 H, d, J = 8 Hz), 7.16 (2 H, d, J = 8 Hz).

### (3) 2-(4-Chlorophenoxymethyl)-1-(4-propylphenyl)piperazine hydrochloride Pale yellow amorphous

¹H NMR (CD₃OD, 400 MHz) δ: 0.91 (3 H, t, J = 7 Hz), 1.5-1.7 (2 H, m), 2.55 (2 H, t, J = 7 Hz), 3.4-3.8 (6 H, m), 3.9-4.1 (2 H, m), 4.2-4.3 (1 H, m), 6.84 (2 H, d, J = 9 Hz), 7.1-7.3 (6 H, m).

### Example 3

### 2-(4-Chlorophenoxymethyl)-1-(3-isopropylphenyl)piperazine hydrochloride

### (1) [4-Benzyl-1-(3-isopropylphenyl)piperazin-2-yl]methanol

¹H NMR (CDCl₃, 400 MHz) δ: 1.22 (6 H, d, J = 7 Hz), 2.35 (1 H, td, J = 4, 11 Hz), 2.5-2.7 (1 H, m), 2.7-2.9 (1 H, m), 2.9-3.0 (1 H, m), 3.12 (1 H, d, J = 11 Hz), 3.3-3.4 (1 H, m), 3.4-3.6 (3 H, m), 3.7-4.0 (3 H, m), 4.1-4.4 (1 H, br s), 6.6-6.8 (3 H, m), 7.17 (1 H, t, J = 8 Hz), 7.2-7.4 (5 H, m).

### (2) 4-Benzyl-2-(4-chlorophenoxymethyl)-1-(3-isopropylphenyl)piperazine

¹H NMR (CDCl₃, 400 MHz) δ: 1.22 (6 H, d, J = 7 Hz), 2.3-2.5 (2 H, m), 2.7-3.0 (2 H, m), 3.1-3.3 (2 H, m), 3.3-3.5 (1H, m), 3.51 (1H, d, J = 13 Hz), 3.60 (1H, d, J = 14 Hz), 3.8-4.0 (1 H, m), 4.0-4.2 (1 H, m), 4.38 (1 H, t, J = 9 Hz), 6.6-6.9 (5 H, m), 7.1-7.3 (8 H, m).

### (3) 2-(4-Chlorophenoxymethyl)-1-(3-isopropylphenyl)piperazine

¹H NMR (CDCl₃, 400 MHz) δ: 1.226 (3 H, d, J = 7 Hz), 1.230 (3 H, d, J = 7 Hz), 2.7-2.9 (1 H, m), 2.9-3.0 (1 H, m), 3.0-3.2 (3 H, m), 3.2-3.4 (2 H, m), 3.87 (1 H, dd, J = 3, 9 Hz), 4.0-4.1 (1H, m), 4.30 (1 H, t, J = 9 Hz), 6.7-6.9 (5 H, m), 7.1-7.3 (3 H, m).

### (4) 2-(4-Chlorophenoxymethyl)-1-(3-isopropylphenyl)piperazine hydrochloride

### Pale brown crystal

¹H NMR (DMSO-d₆, 400 MHz) δ: 1.15 (6 H, d, J = 7 Hz), 2.7-2.9 (1 H, m), 3.0-3.2 (1 H, m), 3.2-3.4 (3 H, m), 3.4-3.5 (1 H, m), 3.5-3.7 (1 H, m), 3.9-4.1 (1 H, m), 4.2-4.4 (2 H, m), 6.7-6.9 (5 H, m), 7.17 (1 H, t, J = 8 Hz), 7.29 (2 H, d, J = 9 Hz), 9.0-9.3 (1 H, br s), 9.4-9.6 (1 H, br s).
IR (Cm⁻¹, KBr): 3437, 2962, 1618, 1597, 1583, 1493, 1464, 1385, 1365, 1342, 1282, 1244, 1171, 1151, 1093, 1043, 1030, 1009, 953, 897, 864, 827, 796, 737, 702, 669, 640, 602, 509, 476.

### Example 4

### 2-(4-Chlorophenoxymethyl)-1-(4-octylphenyl)piperazine hydrochloride

### (1) [4-Benzyl-1-(4-octylphenyl)piperazin-2-yl]methanol

¹H NMR (CDCl₃, 400 MHz) δ: 0.87 (3 H, t, J = 7 Hz), 1.2-1.4 (10 H, m), 1.5-1.7 (2 H, m), 2.36 (1 H, td, J = 4, 11 Hz), 2.51 (2 H, t, J = 8 Hz), 2.5-2.7 (1 H, m), 2.8-3.0 (1 H, m), 3.0-3.2 (1 H, m), 3.2-3.4 (1 H, m), 3.50 (1 H, dd, J = 4, 11 Hz), 3.54 (2 H, s), 3.7-3.9 (2 H, m), 3.8-4.0 (1 H, m), 4.1-4.4 (1 H, br s), 6.82 (2 H, d, J = 9 Hz), 7.06 (2 H, d, J = 8 Hz), 7.2-7.4 (5 H, m).

### (2) 4-Benzyl-2-(4-chlorophenoxymethyl)-1-(4-octylphenyl)piperazine

¹H NMR (CDCl₃, 400 MHz) 5: 0.87 (3 H, t, J = 7 Hz), 1.2-1.4 (10 H, m), 1.5-1.7 (2 H, m), 2.3-2.5 (2 H, m), 2.51 (2 H, t, J = 8 Hz), 2.8-3.0 (1 H, m), 3.0-3.2 (2 H, m), 3.2-3.4 (1 H, m), 3.51 (1 H, d, J = 13 Hz), 3.59 (1 H, d, J = 13 Hz), 3.8-3.9 (1 H, m), 4.0-4.1 (1 H, m), 4.36 (1 H, t, J = 9 Hz), 6.68 (2 H, d, J = 9 Hz), 6.84 (2 H, d, J - 8 Hz), 7.0-7.4 (9 H, m).

### (3) 2-(4-Chlorophenoxymethyl)-1-(4-octylphenyl)piperazine

¹H NMR (CDCl₃, 400 MHz) δ: 0.87 (3 H, t, J = 7 Hz), 1.2-1.4 (10 H, m), 1.5-1.7 (2 H, m), 2.52 (2 H, t, J = 8 Hz), 2.9-3.0 (1 H, m), 3.0-3.2 (3 H, m), 3.2-3.4 (2 H, m), 3.8-3.9 (1 H, m), 3.9-4.1 (1 H, m), 4.26 (1 H, t, J = 9 Hz), 6.73 (2 H, d, J = 9 Hz), 6.86 (2 H, d, J = 9 Hz), 7.07 (2 H, d, J =8 Hz), 7.16 (2 H, d, J = 9 Hz).

### (4) 2-(4-Chlorophenoxymethyl)-1-(4-octylphenyl)piperazine hydrochloride

### Pale yellow crystal

¹H NMR (CD₃OD, 400 MHz) δ: 0.8-1.0 (3 H, m), 1.2-1.4 (10 H, m), 1.5-1.7 (2 H, m), 2.5-2.6 (2 H,m), 3.3-3.7 (6 H, m), 3.9-4.1 (2 H, m), 4.1-4.3 (1 H, m), 6.7-6.9 (2 H, m), 7.1-7.3 (6 H, m). IR (Cm⁻¹, KBr): 3421, 2925, 2854, 1724, 1597, 1516, 1493, 1464, 1408, 1383, 1342, 1284, 1242, 1171, 1149, 1093, 1034, 1009, 958, 928, 868, 825, 764, 706, 669, 617, 553, 509, 474.

### Example 5

### 1-(4-tert-Butylphenyl)-2-(4-chlorophenoxymethyl)piperazine hydrochloride

### (1) 4-Benzyl-1-(4-tert-butylphenyl)-2-(4-chlorophenoxymethyl)piperazine

¹H NMR (CDCl₃, 400 MHz) δ: 1.28 (9 H, s), 2.4-2.5 (2 H, m), 2.89 (1 H, dd, J = 2, 11 Hz), 3.0-3.1 (2 H, m), 3.36 (1 H, dt, J = 3, 11 Hz), 3.51 (1 H, d, J = 13 Hz), 3.59 (1 H, d, J = 13 Hz), 3.87 (1 H, dd, J= 3, 8 Hz), 4.0-4.1 (1 H, m), 4.37 (1 H, t, J = 9 Hz), 6.69 (2 H, d, J = 9 Hz), 6.85 (2 H, d, J = 9 Hz), 7.1-7.2 (2 H, m), 7.2-7.3 (7 H, m).

### (2) 1-(4-tert-Butylphenyl)-2-(4-chlorophenoxymethyl)piperazine

¹H NMR (CDCl₃, 400 MHz) δ: 1.29 (9 H, s), 3.0-3.2 (1 H, m), 3.2-3.4 (4 H, m), 3.49 (1 H, dd, J = 3, 11 Hz), 3.86 (1 H, t, J = 3, 9 Hz), 4.0-4.1 (1 H, m), 4.37 (1 H, t, J = 9 Hz), 6.77 (2 H, d, J = 9 Hz), 6.90 (2 H, d, J = 9 Hz), 7.16 (2 H, d, J = 9 Hz), 7.30 (2 H, d, J = 9 Hz).

### (3) 1-(4-tert-Butylphenyl)-2-(4-chlorophenoxymethyl)piperazine hydrochloride

### Off-white crystal

¹H NMR (CD₃OD, 400 MHz) δ: 1.29 (9 H, s), 3.4-3.7 (6 H, m), 3.9-4.1 (2 H, m), 4.1-4.3 (1 H, m), 6.8-6.9 (2 H, m), 7.1-7.2 (2 H, m), 7.1-7.3 (2 H, m), 7.3-7.5 (2 H, m).
IR (Cm⁻¹, KBr): 2964, 2870, 2501, 1597, 1581, 1516, 1493, 1456, 1423, 1363, 1306, 1286, 1246, 1173, 1155, 1093, 1059, 1043, 1022, 1009, 972, 928, 843, 822, 696, 667, 565, 515, 482.

### Example 6

### 2-(4-Chlorophenoxymethyl)-1-(2,6-dimethylphenyl)piperazine hydrochloride

### (1) 4-Benzyl-2-(4-chlorophenoxymethyl)-1-(2,6-dimethylphenyl)piperazine

¹H NMR (CDC1₃, 400 MHz) δ: 2.31 (3 H, s), 2.36 (3 H, s), 2.3-2.4 (2 H, m), 2.70 (1 H, d, J = 11 Hz), 2.95 (1 H, dt, J = 3, 11 Hz), 3.05 (1 H, d, J = 9 Hz), 3.30 (1 H, td, J = 2, 11 Hz), 3.54 (1 H, d, J = 13 Hz), 3.6-3.7 (3 H, m), 3.7-3.8 (1 H, m), 6.57 (2 H, d, J = 9 Hz), 6.9-7.0 (3 H, m), 7.11 (2 H, d, J = 9 Hz), 7.2-7.4 (5 H, m).

### (2) 2-(4-Chlorophenoxymethyl)-1-(2,6-dimethylphenyl)piperazine

¹H NMR (CDCl₃, 400 MHz) δ: 2.30 (3 H, s), 2.39 (3 H, s), 3.09 (1 H, dt, J = 3, 12 Hz), 3.2-3.3 (2 H, m), 3.47 (1 H, d, J = 12 Hz), 3.6-3.8 (3 H, m), 3.83 (1 H, td, J = 3, 11 Hz), 4.2-4.3 (1 H, m), 6.57 (2 H, d, J = 9 Hz), 6.9-7.0 (1 H, m), 7.0-7.1 (2 H, m), 7.12 (2 H, d, J = 9 Hz).

### (3) 2-(4-Chlorophenoxymethyl)-1-(2,6-dimethylphenyl)piperazine hydrochloride

### White crystal

mp: 97-100°C
¹H NMR (DMSO-d₆, 400 MHz) δ: 2.29 (3 H, s), 2.32 (3 H, s), 2.9-3.0 (1 H, m), 3.0-3.2 (2 H, m), 3.2-3.3 (1 H, m), 3.46 (1 H, d, J = 11 Hz), 3.53 (1 H, td, J = 3, 11 Hz), 3.64 (1 H, dd, J = 5, 11 Hz), 3.78 (1 H, dd, J = 3, 11 Hz), 3.9-4.1 (1 H, m), 6.72 (2 H, d, J = 9 Hz), 6.9-7.0 (3 H, m), 7.23 (2 H, d, J = 9 Hz), 9.3-9.5 (2 H, br s). IR (Cm⁻¹, KBr): 2952, 2924, 2542, 2478, 1597, 1493, 1450, 1398, 1375, 1346, 1281, 1240, 1196, 1161, 1136, 1092, 1034, 1007, 985, 958, 916, 823, 771, 710, 654, 633, 615, 505.

### Example 7

### 2-(4-Chlorophenoxymethyl)-1-(2,4,6-trimethylphenyl)piperazine hydrochloride

### (1) 4-Benzyl-2-(4-chlorophenoxymethyl)-1-(2,4,6-trimethylphenyl)piperazine

¹H NMR (CDCl₃, 400 MHz) 5: 2.21 (3 H, s), 2.27 (3 H, s), 2.32 (3 H, s), 2.3-2.4 (2 H, m), 2.69 (1 H, d, J = 11 Hz), 2.89 (1 H, dt, J = 3, 11 Hz), 3.06 (1 H, d, J = 11 Hz), 3.29 (1 H, td, J = 3,11 Hz), 3.53 (1 H, d, J = 13 Hz), 3.6-3.7 (3 H, m), 3.7-3.8 (1 H, m), 6.59 (2 H, d, J = 9 Hz), 6.73 (1 H, s), 6.80 (1 H, s), 7.11 (2 H, d, J = 9 Hz), 7.2-7.4 (5 H, m).

### (2) 2-(4-Chlorophenoxymethyl)-1-(2,4,6-trimethylphenyl)piperazine

¹H NMR (CDCl₃, 400 MHz) δ: 2.21 (3 H, s), 2.27 (3 H, s), 2.34 (3 H, s), 3.00 (1 H, dt, J = 3, 11 Hz), 3.1-3.2 (2 H, m), 3.33 (1 H, d, J = 11 Hz), 3.5-3.7 (4 H, m), 4.0-4.1 (1 H, m), 6.59 (2 H, d, J = 9 Hz), 6.75 (1 H, s), 6.82 (1 H, s), 7.13 (2 H, d, J = 9 Hz).

### (3) 2-(4-Chlorophenoxymethyl)-1-(2,4,6-trimethylphenyl)piperazine hydrochloride

### Pale brown crystal

¹H NMR (CD₃OD, 400 MHz) 5: 2.18 (3 H, s), 2.31 (3 H, s), 2.32 (3 H, s), 3.09 (1 H, dt, J = 3, 11 Hz), 3.2-3.3 (2 H, m), 3.39 (1 H, d, J = 11 Hz), 3.5-3.7 (2 H, m), 3.69 (1 H, dd, J = 5, 11 Hz), 3.80 (1 H, dd, J = 3, 9 Hz), 3.9-4.1 (1 H, m), 6.6-6.7 (2 H, m), 6.78 (1 H, s), 6.82 (1H, s), 7.1-7.2 (2 H, m).

### Example 8

### 2-(4-Chlorophenoxymethyl)-1-(3,5-di-tert-butylphenyl)piperazine hydrochloride

### (1) [4-Benzyl-1-(di-tert-butylphenyl)piperazin-2-yl]methanol

¹H NMR (CDCl₃, 400 MHz) δ: 1.29 (18 H, s), 2.38 (1 H, td, J = 4, 11 Hz), 2.5-2.7 (1 H, m), 2.8-3.0 (1 H, m), 3.08 (1 H, d, J = 11 Hz), 3.3-3.4 (1 H, m), 3.4-3.6 (3 H, m), 3.7-3.9 (2 H, m), 3.8-4.0 (1 H, m), 4.1-4.3 (1 H, br s), 6.77 (2 H, d, J =2Hz), 6. 94 (1H, s), 7.2-7.4 (5H, m).

### (2) 4-Benzyl-2-(4-chlorophenoxymethyl)-1-(3,5-di-tert-butylphenyl)piperazine

¹H NMR (CDCl₃, 400 MHz) δ: 1.29 (18 H, s), 2.3-2.5 (2 H, m), 2.8-2.9 (1 H, m), 3.0-3.1 (1 H, m), 3.1-3.3 (1 H, m), 3.3-3.4 (1 H, m), 3.53 (1 H, d, J = 13 Hz) 3.60 (1 H, d, J = 13 Hz), 3.8-4.0 (1 H, m), 4.0-4.1 (1 H, m), 4.36 (1 H, t, J = 9 Hz), 6.69 (2 H, d, J = 9 Hz), 6.80 (2 H, d, J = 2 Hz), 6.94 (1 H, s), 7.16 (2 H, d, J = 9 Hz), 7.2-7.4 (5 H, m).

### (3) 2-(4-Chlorophenoxymet hyl)-1-(3,5-di-tert-butylphenyl)piperazine

¹H NMR (CDCl₃, 400 MHz) δ: 1.30 (18 H, s), 2.9-3.1 (1 H, m), 3.0-3.2 (3 H, m), 3.2-3.4 (2 H, m), 3.8-4.0 (2 H, m), 4.27 (1 H, t, J = 9 Hz), 6.74 (2 H, d, J = 9 Hz), 6.82 (2 H, d, J = 2 Hz), 6.96 (1 H, d, J = 1Hz), 7.16 (2 H, d, J = 9 Hz).

### (4) 2-(4-Chlorophenoxymethyl)-1-(3,5-di-tert-butylphenyl)piperazine hydrochloride

### White powder

¹H NMR (CD₃OD, 400 MHz) δ: 1.24 (18 H, s), 3.6-3.9 (6 H, m), 4.0-4.2 (2 H, m), 4.3-4.5 (1 H, m), 6.88 (2 H, d, J = 9 Hz), 7.22 (2 H, d, J = 9 Hz), 7.33 (2 H, s), 7.42 (1 H, s).
IR (Cm⁻¹, KBr): 3431, 2964, 2906, 2870, 2362, 1618, 1595, 1516, 1493, 1466, 1429, 1396, 1365, 1284, 1244, 1171, 1153, 1093, 1032, 1009, 955, 928, 889, 858, 825, 708, 669, 634, 602, 509.

### Example 9

### 2-(4-Chlorophenoxymethyl)-1-(4-cyclohexylphenyl)piperazine hydrochloride

### (1) 4-Benzyl-2-(4-chlorophenoxymethyl)-1-(4-cyclohexylphenyl)piperazine

¹H NMR (CDCl₃, 400 MHz) δ: 1.2-1.3 (1 H, m), 1.3-1.4 (4 H, m), 1.7-1.8 (1 H, m), 1.8-1.9 (4 H, m), 2.33 (1 H, dd, J = 4, 11 Hz), 2.4-2.5 (2 H, m), 2.88 (1 H, dd, J = 2, 11 Hz), 3.0-3.1 (2 H, m), 3.34 (1 H, dt, J = 3, 11 Hz), 3.50 (1 H, d, J = 13 Hz), 3.59 (1 H, d, J = 13 Hz), 3.86 (1 H, dd, J = 3, 8 Hz), 4.0-4.1 (1 H, m), 4.36 (1 H, t, J = 8 Hz), 6.68 (2 H, d, J = 8 Hz), 6.84 (2 H, d, J = 8 Hz), 7.09 (2 H, d, J = 8 Hz), 7.16 (2 H, d, J = 8Hz), 7.2-7.3 (5 H, m).

### (2) 2-(4-Chlorophenoxymethyl)-1-(4-cyclohexylphenyl)piperazine

¹H NMR (CDCl₃, 400 MHz) δ: 1.2-1. (1 H, m), 1.3-1.4 (4 H, m), 1.7-1.8 (1 H, m), 1.8-1.9 (4 H, m), 2.4-2.6 (3 H, m), 2.9-3.0 (1 H, m), 3.1-3.2 (3 H, m), 3.2-3.4 (2 H, m), 3.85 (1 H, dd, J = 3, 8 Hz), 3.9-4.0 (1 H, m), 4.27 (1 H, t, J = 8 Hz), 6.74 (2 H, d, J = 8 Hz), 6.87 (2 H, d, J = 8 Hz), 7.11 (2 H, d, J = 8 Hz), 7.17 (2 H, d, J = 8 Hz).

### (3) 2-(4-Chlorophenoxymethyl)-1-(4-cyclohexylphenyl)piperazine hydrochloride

### Pale brown crystal

¹H NMR (CD₃OD, 400 MHz) δ: 1.2-1.5 (5 H, m), 1.7-1.9 (5 H, m), 2.4-2.5 (1 H, m), 3.4-3.7 (6 H, m), 4.01 (2 H, d, J = 5 Hz), 4.1-4.2 (1 H, m), 6.83 (2 H, d, J = 9 Hz), 7.1-7.2 (2 H, m), 7.2-7.3 (4 H, m).

### Example 10

### 2-(4-Chlorophenoxymethyl)-1-indan-5-yl-piperazine hydrochloride

### (1) (4-Benzyl-1-indan-5-y1-piperazin-2-yl)methanol

¹H NMR (CDCl₃, 400 MHz) δ: 2.0-2.1 (2 H, m), 2.37 (1 H, td, J = 4, 11 Hz), 2.6-2.7 (1 H, m), 2.8-3.0 (5 H, m), 3.0-3.1 (1 H, m), 3.27 (1 H, td, J = 3, 6 Hz), 3.50 (1 H, dd, J = 4, 11 Hz), 3.54 (2 H, s), 3.7-3.8 (2 H, m), 3.8-3.9 (1 H, m), 6.71 (1 H, dd, J = 2,8 Hz), 6.82 (1 H, s), 7.09 (1 H, d, J = 8 Hz), 7.2-7.4 (5H,m).

### (2) 4-Benzyl-2-(4-chlorophenoxymethyl)-1-indan-5-yl-piperazine

¹H NMR (CDCl₃, 400 MHz) δ: 2.0-2.1 (2 H, m), 2.36 (1 H, td, J = 3, 11 Hz), 2.43 (1 H, dd, J = 3, 11 Hz), 2.8-2.9 (5 H, m), 3.0-3.1 (1H, m), 3.16 (1 H, td, J = 3, 11 Hz), 3.2-3.4 (1 H, m), 3.51 (1 H, d, J = 13 Hz), 3.60 (1 H, d, J = 13 Hz), 3.85 (1 H, dd, J = 3, 9 Hz), 4.0-4.1 (1 H, m), 4.36 (1 H, t, J = 9 Hz), 6.69 (2 H, d, J = 9 Hz), 6.72 (1 H, dd, J = 2, 8Hz), 6.8-6.9 (1 H, br s), 7.10 (1 H, d, J = 8 Hz), 7.16 (2 H, d, J = 9 Hz), 7.2-7.3 (5 H,m) .

### (3) 2-(4-Chlorophenoxymethyl)-1-indan-5-yl-piperazine

¹H NMR (CDCl₃, 400 MHz) δ: 2.0-2.1 (2 H, m), 2.8-3.3 (10 H, m), 3.84 (1 H, dd, J = 4, 9 Hz), 3.9-4.0 (1 H, m), 4.24 (1 H, t, J = 9 Hz), 6.74 (2 H, d, J = 9Hz), 6.7-6.8 (1 H, m), 6.8-6.9 (1H, br s), 7.10 (1 H, d, J = 8 Hz), 7.16 (2 H, d, J = 9 Hz).

### (4) 2-(4-Chlorophenoxymethyl)-1-indan-5-yl-piperazine hydrochloride

### White crystal

¹H NMR (CD₃OD, 400 MHz) δ: 2.0-2.2 (2 H, m), 2.8-2.9 (4 H, m), 3.5-3.8 (6 H, m), 3.9-4.0 (1 H, m), 4.0-4.1 (1 H, m), 4.2-4.3 (1 H, br s); 6.85 (2 H, d, J = 9 Hz), 7.1-7.2 (1 H, m), 7.22 (2 H, d, J = 9 Hz), 7.2-7.3 (2 H, m).

### Example 11

### 1-(4-Isopropylphenyl)-2-(3,4-methylenedioxyphenoxymethyl)piperazine hydrochloride

### (1) 4-Benzyl-1-(4-isopropylphenyl)-2-(3,4-methylenedioxyphenoxymethyl)piperazine

¹H NMR (CDCl₃, 400 MHz) δ: 1.21 (6 H, d, J = 7 Hz), 2.32 (1 H, td, J = 3, 11 Hz), 2.40 (1 H, dd, J = 3,11 Hz), 2.7-2.9 (2 H, m), 3.0-3.2 (2 H, m), 3.2-3.4 (1 H, m), 3.52 (1 H, d, J = 13 Hz), 3.58 (1 H, d, J = 14 Hz), 3.83 (1 H, dd, J = 4, 9 Hz), 4.0-4.1 (1 H, m), 4.31 (1 H, t, J = 9 Hz), 5.8-5.9 (2 H, m), 6.20 (1 H, dd, J = 2, 8Hz), 6.34 (1 H, d, J = 2 Hz), 6.64 (1 H, d, J = 8 Hz), 6.85 (2 H, d, J = 9 Hz), 7.11 (2 H, d, J = 9 Hz), 7.1-7.4 (5 H, m).

### (2) 1-(4-Isopropylphenyl)-2-(3,4-methylenedioxyphenoxymethyl)piperazine

¹H NMR (CDCl₃, 400 MHz) δ: 1.22 (6 H, d, J = 7 Hz), 2.7-2.9 (1 H, m), 2.9-3.0 (1 H, m), 3.0-3.2 (3 H, m), 3.2-3.4 (2 H, m), 3.7-3.9 (1 H, m), 3.9-4.0 (1 H, m), 4.21 (1 H, t, J = 9 Hz), 5.88 (2 H, s), 6.23 (1 H, dd, J = 2, 8 Hz), 6.41 (1 H, d, J = 2 Hz), 6.64 (1 H, d, J = 9 Hz), 6.87 (2 H, d, J = 9 Hz), 7.12 (2 H, d, J = 9 Hz).

### (3) 1-(4-Isopropylphenyl)-2-(3,4-methylenedioxyphenoxymethyl)piperazine hydrochloride

### White powder

¹H NMR (DMSO-d₆, 400 MHz) δ: 1.16 (6 H, d, J = 7 Hz), 2.7-2.9 (1 H, m), 3.0-3.2 (1 H, m), 3.2-3.4 (3 H, m), 3.3-3.6 (2 H, m), 3.8-4.0 (1 H, m), 4.1-4.3 (2 H, m), 5.93 (2 H, s), 6.29 (1 H, dd, J = 2, 8 Hz), 6.55 (1 H, d, J = 2 Hz), 6.75 (1 H, d, J = 9 Hz), 6.94 (2 H, d, J = 9 Hz), 7.13 (2 H, d, J = 8 Hz), 9.0-9.2 (1 H, br s), 9.4-9.6 (1 H, br s).

### Example 12

### 2-(4-Benzyloxyphenoxymethyl)-1-(4-isopropylphenyl)piperazine hydrochloride

### (1) 4-Benzyl-2-(4-benzyloxyphenoxymethyl)-1-(4-isopropylphenyl)piperazine

¹H NMR (CDCl₃, 400 MHz) δ: 1.21 (6 H, d, J = 7 Hz), 2.2-2.5 (2 H, m), 2.7-2.9 (2 H, m), 3.1-3.2 (2 H,m), 3.2-3.4 (1 H, m), 3.53 (1 H, d, J = 14Hz), 3.58 (1 H, d, J = 13 Hz), 3.8-3.9 (1 H, m), 4.0-4.1 (1 H, m), 4.34 (1 H, t, J = 9 Hz), 5.00 (2 H, s), 6.6-6.8 (2 H, m), 6.8-6.9 (4 H, m), 7.0-7.5 (12 H, m).

### (2) 2-(4-Benzyloxyphenoxymethyl)-1-(4-isopropylphenyl)piperazine

¹H NMR (CDCl₃, 400 MHz) δ: 1.22 (6 H, d, J = 7 Hz), 2.7-2.9 (1 H, m), 2.9-3.0 (1 H, m), 3.0-3.2 (3 H, m), 3.2-3.4 (2 H, m), 3.83 (1 H, dd, J = 4, 9 Hz), 3.9-4.0 (1 H, m), 4.23 (1 H, t, J =9 Hz), 4.99 (2 H, s), 6.7-6.8 (2 H, m), 6.8-6.9 (4 H, m), 7.11 (2 H, d, J = 9 Hz), 7.2-7.5 (5 H, m).

### (3) 2-(4-Benzyloxyphenoxymethyl)-1-(4-isopropylphenyl)piperazine hydrochloride

### White powder

¹H NMR (DMSO-d₆, 400 MHz) δ: 1.16 (6 H, d, J = 7 Hz), 2.7-2.9 (1 H, m), 3.0-3.2 (1 H, m), 3.2-3.4 (3 H, m), 3.4-3.6 (2 H, m), 3.8-3.9 (1 H, m), 4.2-4.3 (2 H, m), 5.01 (2 H, s), 6.79 (2 H, d, J = 9 Hz), 6.89 (2 H, d, J = 9 Hz), 6.96 (2 H, d, J = 9 Hz), 7.13 (2 H, d, J = 8 Hz), 7.2-7.5 (5 H, m), 9.2 (1 H, br s), 9.6 (1 H, br s). IR (Cm⁻¹, KBr): 2960, 1639, 1610, 1593, 1508, 1454, 1383, 1344, 1304, 1228, 1149, 1109, 1080, 1038, 958, 914, 866, 825, 796, 777, 739, 696, 663, 600, 567, 523, 469.

### Example 13

### 2-(4-tert-Butoxyphenoxymethyl)-1-(3-isopropylphenyl)piperazine hydrochloride

### (1) 4-Benzyl-2-(4-tert-Butoxyphenoxymethyl)-1-(3-isopropylphenyl)piperazine

¹H NMR (CDC1₃, 400 MHz) δ: 1.220 (3 H, d, J = 7 Hz), 1.230 (3 H, d, J = 7 Hz), 1.29 (9 H, s), 2.2-2.5 (2 H, m), 2.7-3.0 (2 H, m), 3.1-3.3 (2 H, m), 3.3-3.5 (1 H, m), 3.54 (1 H, d, J = 13 Hz), 3.59 (1 H, d, J = 14 Hz), 3.87 (1 H, dd, J = 3, 9 Hz), 4.0-4.2 (1 H, m), 4.38 (1 H, t, J = 9 Hz), 6.6-6.9 (7 H, m), 7.1-7.4 (6 H, m).

### (2) 2-(4-tert-Butoxyphenoxymethyl)-1-(3-isopropylphenyl)piperazine

¹H NMR (CDCl₃, 400 MHz) δ: 1.226 (3 H, d, J = 7 Hz), 1.230 (3 H, d, J = 7 Hz), 1.28 (9 H, s), 2.7-2.9 (1 H, m), 2.9-3.0 (1 H, m), 3.0-3.2 (3 H, m), 3.2-3.4 (2 H, m), 3.86 (1 H, dd, J = 3, 9 Hz), 4.0-4.1 (1 H, m), 4.28 (1 H, t, J = 9 Hz), 6.6-6.9 (7 H, m), 7.18 (1 H, t, J = 8 Hz).

### (3) 2-(4-tert-Butoxyphenoxymethyl)-1-(3-isopropylphenyl)piperazine hydrochloride White powder

¹H NMR (DMSO-d₆, 400 MHz) δ: 1.15 (6 H, d, J = 7 Hz), 1.21 (9 H, s), 2.7-2.9 (1 H, m), 3.0-3.2 (1 H, m), 3.2-3.4 (3 H, m), 3.4-3.5 (1 H, m), 3.5-3.7 (1 H, m), 3.9-4.0 (1 H, m), 4.2-4.4 (2 H, m), 6.7-6.9 (7 H, m), 7.17 (1 H, t, J = 8 Hz), 9.1-9.3 (1 H, br s), 9.6-9.8 (1 H, br s). IR (Cm⁻¹, KBr): 3419, 2974, 1601, 1504, 1462, 1389, 1365, 1344, 1281, 1223, 1169, 1103, 1082, 1032, 957, 930, 897, 849, 833, 796, 737, 700, 660, 638, 571, 523, 474.

### Example 14

### 2-(4-Cyanophenoxymethyl)-1-(3-isopropylphenyl)piperazine hydrochloride

### (1) 4-Benzyl-2-(4-cyanophenoxymethyl)-1-(3-isopropylphenyl)piperazine

¹H NMR (CDCl₃, 400 MHz) δ: 1.219 (3 H, d, J = 7 Hz), 1.224 (3 H, d, J = 7 Hz), 2.3-2.5 (2 H, m), 2.7-3.0 (2 H, m), 3.0-3.2 (1 H, m), 3.1-3.3 (1 H, m), 3.3-3.5 (1 H, m), 3.50 (1 H, d, J = 14 Hz), 3.61 (1 H, d, J = 13 Hz), 3.96 (1 H, dd, J = 4, 9 Hz), 4.1-4.2 (1 H, m), 4.46 (1 H, t, J = 9 Hz), 6.7-6.9 (5 H, m), 7.1-7.3 (6 H, m), 7.4-7.6 (2 H, m).

### (2) 2-(4-Cyanophenoxymethyl)-1-(3-isopropylphenyl)piperazine

¹H NMR (CDC1₃, 400 MHz) δ: 1.225 (3 H, d, J = 7 Hz), 1.229 (3 H, d, J = 7 Hz), 2.7-2.9 (1 H, m), 2.9-3.0 (1 H, m), 3.0-3.2 (3 H, m), 3.2-3.4 (2 H, m), 3.95 (1 H, dd, J = 4, 9 Hz), 4.0-4.2 (1 H, m), 4.40 (1 H, t, J = 9 Hz), 6.7-6.9 (5 H, m), 7.19 (1 H, t, J = 8 Hz), 7.4-7.6 (2 H, m).

### (3) 2-(4-Cyanophenoxymethyl)-1-(3-isopropylphenyl)piperazine hydrochloride Pale brown powder

¹H NMR (CD₃OD, 400 MHz) δ: 1.14 (3 H, d, J = 7 Hz), 1.17 (3 H, d, J = 7 Hz), 2.7-2.9 (1 H, m), 3.4-3.6 (3 H, m), 3.6-3.7 (3 H, m), 4.0-4.2 (2 H, m), 4.2-4.3 (1 H, m), 6.9-7.1 (5 H, m), 7.28 (1 H, t, J = 8 Hz), 7.5-7.7 (2 H, m) .
IR (Cm⁻¹, KBr): 3423, 2962, 2771, 2225, 1728, 1604, 1577, 1508, 1462, 1387, 1342, 1304, 1255, 1174, 1151, 1113, 1080, 1045, 1028, 957, 928, 839, 798, 737, 702, 640, 550, 515, 476.

### Example 15

### 1-(4-Isopropylphenyl)-2-(4-sulfamoylphenoxymethyl)piperazine hydrochloride

### (1) 4-Benzyl-1-(4-isopropylphenyl)-2-(4-sulfamoylphenoxymethyl)piperazine

¹H NMR (CDCl₃, 400 MHz) 5: 1.23 (6 H, d, J = 7 Hz), 2.3-2.5 (2 H, m), 2.7-3.0 (2 H, m), 3.0-3.3 (2 H, m), 3.3-3.4 (1 H, m), 3.50 (1 H, d, J = 13 Hz), 3.60 (1 H, d, J = 13 Hz), 3.96 (1 H, dd, J = 4, 9 Hz), 4.0-4.2 (1 H, m), 4.46 (1 H, t, J = 9 Hz), 4.67 (2 H, s), 6.8-6.9 (4 H, m), 7.12 (2 H, d, J = 9 Hz), 7.1-7.3 (5 H, m), 7.79 (2 H, d, J = 9 Hz).

### (2) 1-(4-Isopropylphenyl)-2-(4-sulfamoylphenoxymethyl)piperazine

¹H NMR (DMSO-d₆, 400 MHz) 5: 1.15 (6 H, d, J = 7 Hz), 2.6-2.9 (2 H, m), 2.8-3.0 (3 H, m), 3.10 (1 H, d, J = 7 Hz), 3.1-3.3 (1 H, m), 3.81 (1 H, dd, J = 4, 9 Hz), 3.9-4.1 (1 H, m), 4.43 (1 H, t, J = 9 Hz), 6.85 (2 H, d, J = 9 Hz), 6.98 (2 H, d, J = 9 Hz), 7.07 (2 H, d, J = 8 Hz), 7.16 (2 H, s), 7.68 (2 H, d, J = 9 Hz).

### (3) 1-(4-Isopropylphenyl)-2-(4-sulfamoylphenoxymethyl)piperazine hydrochloride

### White powder

¹H NMR (DMSO-d₆, 400 MHz) δ: 1.16 (6 H, d, J = 7 Hz), 2.7-2.9 (1 H, m), 3.0-3.2 (1 H, m), 3.2-3.4 (3 H, m), 3.4-3.6 (2 H, m), 4.0-4.1 (1 H, m),4.2-4.5 (2 H, m), 6.9-7.1 (4 H, m), 7.13 (2 H, d, J = 9 Hz), 7.2 (2 H, br s), 7.70 (2 H, d, J = 9 Hz), 9.1 (1 H, br s), 9.5 (1 H, br s). IR (Cm⁻¹, KBr): 3053, 2960, 2871, 2679, 2465, 2355, 1595, 1516, 1500, 1460, 1412, 1387, 1331, 1304, 1254, 1157, 1099, 1030, 957, 912, 835, 725, 692, 642, 627, 586, 569, 548, 513, 407.

### Example 16

### 1-(4-Isopropylphenyl)-2-[4-(isopropylsulfamoyl)phenoxymethyl]piperazine hydrochloride

### (1) 4-Benzyl-1-(4-isopropylphenyl)-2-[4-(isopropylsulfamoyl)phenoxymethyl]piperazine

¹H NMR (CDCl₃, 400 MHz) 5: 1. 07 (6 H, d, J = 6 Hz), 1.21 (6 H, d, J = 7 Hz), 2.3-2.5 (2 H, m), 2.7-3.0 (2 H, m), 3.0-3.3 (2 H, m), 3.3-3.5 (2 H, m), 3.50 (1 H, d, J = 13 Hz), 3.61 (1 H, d, J = 14 Hz), 3.95 (1 H, dd, J = 5, 8 Hz), 4.0-4.2 (1 H, m), 4.29 (1 H, d, J = 7 Hz), 4.46 (1 H, t, J = 9 Hz), 6.8-6.9 (4 H, m), 7.12 (2 H, d, J = 8 Hz), 7.1-7.3 (5 H, m), 7.75 (2 H, d, J = 9Hz).

### (2) 1-(4-Isopropylphenyl)-2-[4-(isopropylsulfamoyl)phenoxymethyl]piperazine

¹H NMR (CDCl₃, 400 MHz) δ: 1.05 (6 H, d, J = 6 Hz), 1.22 (6 H, d, J = 7 Hz), 2.7-2.9 (1 H, m), 2.9-3.0 (1 H, m), 3.0-3.2 (3 H, m), 3.2-3.4 (2 H, m), 3.3-3.5 (1 H, m), 3.93 (1 H, dd, J = 4,9 Hz), 4.0-4.1 (1 H, m), 4.3-4.4 (1 H, br s), 4.38 (1 H, t, J = 9 Hz), 6.8-7.0 (4 H, m), 7.13 (2 H, d, J = 8 Hz), 7.74 (2 H, d, J = 9 Hz).

### (3) 1-(4-Isopropylphenyl)-2-[4-(isopropylsulfamoyl)phenoxymethyl]piperazine hydrochloride

### White powder

¹H NMR (DMSO-d₆, 400 MHz) δ: 0.92 (6 H, d, J = 6 Hz), 1.16 (6 H, d, J = 7 Hz), 2.7-2.9 (1 H, m), 3.0-3.2 (2 H, m), 3.2-3.4 (3 H, m), 3.4-3.6 (2 H, m), 4.06 (1 H, dd, J = 5, 10 Hz), 4.2-4.5 (2 H, m), 6.96 (2 H, d, J = 9 Hz), 7.02 (2 H, d, J = 9 Hz), 7.12 (2 H, d, J = 9 Hz), 7.40 (1 H, d, J = 7 Hz), 7.68 (2 H, d, J = 9 Hz), 9.2 (1 H, br s), 9.6 (1 H, br s). IR (Cm⁻¹, KBr): 2966, 2933, 2873, 2681, 2362, 1633, 1595, 1516, 1498, 1464, 1427, 1387, 1323, 1300, 1254, 1157, 1093, 1016, 1001, 958, 893, 837, 717, 690, 642, 627, 588, 569.

### Example 17

### 2-(4-Chlorophenylsulfanylmethyl)-1-(4-isopropylphenyl)piperazine hydrochloride

### (1) 4-Benzyl-2-(4-chlorophenylsulfanylmethyl)-1-(4-isopropylphenyl)piperazine

¹H NMR (CDC1₃, 400 MHz) δ: 1.225 (3 H, d, J = 7 Hz), 1.228 (3 H, d, J = 7 Hz), 2.25 (1 H, dt, J = 4, 11 Hz), 2.3-2.4 (1 H, m), 2.8-2.9 (3 H, m), 3.0-3.3 (3 H, m), 3.3-3.6 (3 H, m), 3.6-3.8 (1 H, m), 6.64 (2 H, d, J = 9 Hz), 7.0-7.2 (5 H, m), 7.2-7.4 (6 H, m).

### (2) 2-(4-Chlorophenylsulfanylmethyl)-1-(4-isopropylphenyl)piperazine

¹H NMR (CDCl₃, 400 MHz) δ: 1.23 (3 H, d, J = 7 Hz), 1.24 (3 H, d, J = 7 Hz), 2.8-3.0 (3 H, m), 3.0-3.2 (4 H, m), 3.34 (1 H, dd, J = 3, 13 Hz), 3.3-3.4 (1 H, m), 3.6-3.7 (1 H, m), 6.67 (2 H, d, J = 9 Hz), 7.08 (2 H, d, J = 9 Hz), 7.1-7.2 (4 H, m).

### (3) 2-(4-Chlorophenylsulfanylmethyl)-1-(4-isopropylphenyl)piperazine hydrochloride

### Pale brown amorphous

¹H NMR (DMSO-d₆, 400 MHz) δ: 1.157 (3 H, d, J = 7 Hz), 1.162 (3 H, d, J = 7 Hz), 2.7-2.9 (1 H, m), 2.91 1 (1 H, dd, J = 3, 14 Hz), 3.0-3.1 (1 H, m), 3.1-3.3 (3 H, m), 3.3-3.6 (3 H, m), 3.8-3.9 (1 H, m), 6.71 (2 H, d, J = 9 Hz), 7.07 (2 H, d, J = 9 Hz), 7.2-7.4 (4 H, m), 9.3-9.4 (2 H, br).

### Example 18

### 2-(4-Hydroxyphenoxymethyl)-1-(4-isopropylphenyl)piperazine hydrochloride

### (1) 2-(4-Hydroxyphenoxymethyl)-1-(4-isopropylphenyl)piperazine

To a solution of 4-benzyl-2-(4-benzyloxyphenoxymethyl)-1-(4-isopropylphenyl)piperazine (320 mg, 0.632 mmol, obtained in Example 12 (1)) in methanol (10 mL) was added acetic acid (0.5 mL). The mixture was hydrogenated overnight using 10% palladium-carbon (30 mg) as a catalyst. After removal of the catalyst by filtration, the filtrate was concentrated to dryness, and the residue was partitioned between ethyl acetate and aqueous sodium hydrogen carbonate solution. The collected ethyl acetate layer was washed with saturated brine, dried over sodium sulfate, and evaporated under reduced pressure. The residue was purified by silica gel column chromatography (chloroform/methanol =20/1), to give the titled compound as a pale greenish powder (86 mg, yield 42%).
¹H NMR (CD₃OD, 400 MHz) δ: 1.21 (6 H, d, J = 7 Hz), 2.7-3.0 (2 H, m), 3.0-3.3 (5 H, m), 3.7-3.9 (2 H, m), 4.07 (1 H, t, J = 9 Hz), 6.63 (4 H, s), 6.94 (2 H, d, J = 9 Hz), 7.12 (2 H, d, J = 9 Hz).

### (2) 2-(4-Hydroxyphenoxymethyl)-l-(4-isopropylphenyl)piperazine hydrochloride

The titled compound was obtained as a white powder by the similar manner as described in Example 1 (7).
¹H NMR (CD₃OD, 400 MHz) δ: 1.23 (6 H, d, J = 7 Hz), 2.8-3.0 (1 H, m), 3.4-3.8 (6 H, m), 3.8-4.0 (2 H, m), 4.1-4.3 (1 H, m), 6.6-6.8 (4 H, m), 7.1-7.3 (4 H, m).
IR (Cm⁻¹, KBr): 2960, 2873, 2794, 2679, 2519, 1610, 1510, 1456, 1383, 1365, 1298, 1228, 1217, 1149, 1101, 1082, 1034, 957, 916, 868, 829, 800, 773, 723, 654, 588, 567, 519, 469.

### Example 19

### Pharmacological Experiments

### (Experimental procedures 1)

### 1. P2X₄ antagonism study by measurement of intracellular Ca²⁺ influx response

1321N1 cells stably expressing human P2X₄ receptors were adopted for calcium influx assay. P2X4/1321N1 cells were plated in 96-well assay plate and cultured 24 hours in an atmosphere of 5 % CO₂ at 37°C. Fura-2 AM calcium indicator in assay buffer (150 mM NaCl, 5 mM KC1, 1.8 mM CaCl₂, 1.2 mM MgCl₂, 10 mM D-glucose and 25 mM HEPES, pH7.4) was loaded onto cells for 1 hour at room temperature and the fluorescence was detected by FLUOstar Optima micro plate reader (BMG labtech). The cells were alternatively illuminated with two excitations wavelengths (340 nm and 380 nm) via xenon lamp and the emitted fluorescence was measured at 510 nm. The fluorescence changes after the treatment of 1 µM ATP were monitored and determined the fluorescence ratio (F₃₄₀/F₃₈₀) as the index of intracellular calcium change. Tested compound were treated to cells 15 min before the addition of ATP and the inhibition activity of compounds was calculated by comparing the Ca²⁺ response with control in the absence of tested compound.

### (Experimental procedures 2)

### 2. [³H]paroxetine binding to Serotonin transporter

To investigate the effects of compounds on human Serotonin transporter, [³H]paroxetine competition binding study was performed. Cell membranes prepared from HEK293 cells stably expressing the human Serotonin transporter (1 mg protein/ml) were incubated with 0.4 nM [³H]paroxetine in the presence of tested compound in incubation buffer containing 50 mM Tris-HCl, pH7.4, 120 mM NaCl and 5 mM KCl. After incubation for 60 min at 25°C, the reaction was terminated by rapid filtration through GF/B glass fiber filter and rinsed with ice-cold incubation buffer. The retained radioactivity on filter was measured by a liquid scintillation counter. Nonspecific binding was defined with 10 µM imipramine and subtracted from total binding to determine specific binding. Inhibition activities of tested compounds were determined as a percent inhibition of the specific binding.

### (Experimental procedures 3)

### 3. Serotonin reuptake inhibition assay

To test the serotonin reuptake inhibitory activity of compounds, experiments were carried out as described by Provic and Muller (Arzneim-Forsch. Drug Res., 1995, 45:1145-1148.). 100 µg of the synaptosomes prepared from the rat brain were incubated for 15 min at 37°C with 0.1 µCi [³H]serotonin in the presence of tested compound in a buffer containing 106.2 mM NaCl, 4.5 mM KCl, 2.25 mM MgSO₄, 1.08 mM NaH₂PO₄, 22.5 mM NaHCO₃, 9.9 mM glucose, 9 µM EGTA and 45 µM ascorbic acid (pH 7.4). Basal control activity was determined by incubating the same mixture for 15 min at 4°C in the presence of 10 µM imipramine. Following incubation, the samples were filtered rapidly under vacuum through GF/B glass fiber filters and rinsed twice with ice-cold incubation buffer using a cell harvester. The retained radioactivity on filters was measured in TopCount scintillation counter. The results were expressed as a percent inhibition of the specific binding in the absence of tested compound.

### (Experimental results)

The experimental results obtained by the Experimental procedures 1 are set forth in Table 11. Furthermore, the experimental results obtained by the Experimental procedures 2 and 3 are set forth in Table 12 and 13, respectively.

**TABLE 11**

| Test compound | Inhibitory activity: IC₅₀ (µM) |
|---|---|
| Example 1 | 6.9 |
| Example 2 | 7.6 |
| Example 3 | 8.3 |
| Example 7 | 3.1 |
| Example 10 | 4.7 |
| Example 11 | 7.4 |
| Example 13 | 2.9 |
| Example 15 | 6.3 |
| Example 16 | 2.8 |
| Example 17 | 8.1 |
| Paroxetine | 4.7 |

As is evident from Table 11, the compounds of the formula (I) have excellent P2X₄ receptor antagonism.

**TABLE 12**

| Test compound | Percent inhibition (10⁻⁷ M) |
|---|---|
| Example 1 | 17 |
| Example 2 | 31 |
| Example 3 | 13 |
| Example 7 | 26 |
| Example 10 | 44 |
| Paroxetine | 93 |

As is evident from Table 12, the compounds of the formula (I) have weak binding affinity to serotonin transporter.

**TABLE 13**

| Test compound | Percent inhibition (10⁻⁷ M) |
|---|---|
| Example 16 | 11 |
| Example 17 | 4 |
| Paroxetine | 97 |

As is evident from Table 13, the compounds of the formula (I) have weak inhibitory activity to serotonin reuptake.

## Claims

1. A compound represented by the formula (I) or its pharmacologically acceptable salt: 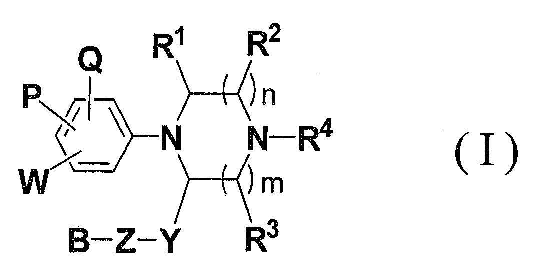 wherein B represents an aryl group or a heterocyclic group, each of which can have a substituent;
Y represents alkylene having 1 to 5 carbon atoms, which can have a double bond;
Z represents O, S, N(R⁵), or a chemical bond, wherein R⁵ represents a hydrogen atom or an alkyl group having 1 to 8 carbon atoms;
each of R¹, R², and R³ independently represents a hydrogen atom, an alkyl group having 1 to 8 carbon atoms, or a haloalkyl group having 1 to 8 carbon atoms and 1 to 3 halogen atoms;
R⁴ represents a hydrogen atom, an alkyl group having 1 to 8 carbon atoms, a haloalkyl group having 1 to 8 carbon atoms and 1 to 3 halogen atoms, a three- to seven-membered cycloalkyl group, or an alkyl group having 1 to 8 carbon atoms substituted with a three- to seven-membered cycloalkyl;
each of P and Q independently represents a hydrogen atom, a halogen atom, a alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, or a heterocyclic group;
W represents an alkyl group having 1 to 8 carbon atoms or a three- to seven-membered cycloalkyl group, or P and W are combined to form propylene or tetramethylene in the case that P and W are placed at the 2- and 3-positions or the 3- and 4-positions of the phenyl group; and
each of n and m independently represents 1 or 2.

2. The compound or its pharmacologically acceptable salt as defined in claim 1, wherein B represents phenyl, naphthyl, benzofuranyl, indolyl, benzothienyl, or thienyl, each of which can have one to three substituent groups selected from the group consisting of a halogen atom, an alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, an aryloxy group having 6 to 12 carbon atoms, an arylalkyloxy group comprising an alkyl moiety having 1 to 8 carbon atoms, an alkoxycarbonyl group having 2 to 9 carbon atoms, a carbamoyl group, an alkylcarbamoyl group having 2 to 9 carbon atoms, a sulfamoyl group, an alkylsulfamoyl group having 1 to 8 carbon atoms, and a dialkylsulfamoyl group having 2 to 16 carbon atoms.

3. The compound or its pharmacologically acceptable salt as defined in claim 1, wherein B represents phenyl, which can have one to three substituent groups selected from the group consisting of a halogen atom, an alkyl group having 1 to 8 carbon atoms, a haloalkyl group having 1 to 8 carbon atoms and 1 to 3 halogen atoms, a nitro group, a cyano group, a hydroxyl group, an amino group, an alkylamino group having 1 to 8 carbon atoms, a dialkylamino group having 2 to 16 carbon atoms, an acylamino group having 2 to 8 carbon atoms, an alkoxy group having 1 to 8 carbon atoms, a haloalkoxy group having 1 to 8 carbon atoms and 1 to 3 halogen atoms, an aryloxy group having 6 to 12 carbon atoms, an arylalkyloxy group comprising an alkyl moiety having 1 to 8 carbon atoms, an alkoxycarbonyl group having 2 to 9 carbon atoms, a carbamoyl group, an alkylcarbamoyl group having 2 to 9 carbon atoms, a sulfamoyl group, an alkylsulfamoyl group having 1 to 8 carbon atoms, and a dialkylsulfamoyl group having 2 to 16 carbon atoms.

4. The compound or its pharmacologically acceptable salt as defined in one of claims 1 to 3, wherein each of P and Q independently represents a hydrogen atom, a alkyl group having 1 to 8 carbon atoms, a haloalkyl group having 1 to 8 carbon atoms and 1 to 3 halogen atoms, or an alkoxy group having 1 to 8 carbon atoms.

5. The compound or its pharmacologically acceptable salt as defined in one of claims 1 to 3, wherein each of P and Q represents a hydrogen atom.

6. The compound or its pharmacologically acceptable salt as defined in one of claims 1 to 5, wherein W represents an alkyl group having 3 to 6 carbon atoms.

7. The compound or its pharmacologically acceptable salt as defined in one of claims 1 to 5, wherein W represents n-propyl, isopropyl, n-butyl, or isobutyl.

8. The compound or its pharmacologically acceptable salt as defined in one of claims 1 to 7, wherein each of n and m represents 1.

9. The compound or its pharmacologically acceptable salt as defined in one of claims 1 to 8, wherein Y represents methylene.

10. The compound or its pharmacologically acceptable salt as defined in one of claims 1 to 9, wherein Z represents O or S.

11. The compound or its pharmacologically acceptable salt as defined in one of claims 1 to 10, wherein each of R¹, R², and R³ represents a hydrogen atom.

12. The compound or its pharmacologically acceptable salt as defined in one of claims 1 to 11, wherein R⁴ represents a hydrogen atom or an alkyl group having 1 to 8 carbon atoms.

13. The compound or its pharmacologically acceptable salt as defined in one of claims 1 to 11, wherein R⁴ represents a hydrogen atom.

14. The compound or its pharmacologically acceptable salt as defined in claim 1, wherein R⁹ represents a hydrogen atom, Y represents methylene, Z represents O or S, and B represents phenyl, which can have one to three substituent groups selected from the group consisting of a halogen atom, an alkyl group having 1 to 8 carbon atoms, a haloalkyl group having 1 to 8 carbon atoms and 1 to 3 halogen atoms, a cyano group, a hydroxyl group, an alkoxy group having 1 to 8 carbon atoms, a haloalkoxy group having 1 to 8 carbon atoms and 1 to 3 halogen atoms, a benzyloxy group, a sulfamoyl group, and an alkylsulfamoyl group having 1 to 8 carbon atoms.

15. A compound selected from the group consisting of 2-(4-chlorophenoxymethyl)-1-(4-isopropylphenyl)piperazine, 2-(4-chlorophenoxymethyl)-1-(4-propylphenyl)piperazine, 2-(4-chlorophenoxymethyl)-1-(3-isopropylphenyl)piperazine, 2-(4-chlorophenoxymethyl)-1-(2,4,6-trimethylphenyl)piperazine, 2-(4-chlorophenoxymethyl)-(1-indan-5-yl)-piperazine, 1-(4-isopropylphenyl)-2-[4-(isopropylsulfamoyl)phenoxymethyl)piperazine, 2-(4-chlorophenylsulfanylmethyl)-1-(4-isopropylphenyl)-piperazine, 1-(3-isopropylphenyl)-2-[4-(isopropylsulfamoyl)phenoxymethyl]piperazine, and 1-(4-isopropylphenyl)-2-(4-phenoxyphenoxymethyl)piperazine or its pharmacologically acceptable salt.

16. A P2X₄ receptor antagonist containing a compound defined in any one of claims 1 to 15 or its pharmacologically acceptable salt as an active ingredient.

17. A preventive or therapeutic agent for neuropathic pains containing a compound defined in any one of claims 1 to 15 or its pharmacologically acceptable salt as an active ingredient.
